(19) Europäisches Patentamt
European Patent Office
Office européen des brevets

(11) Numéro de publication: **0 551 230 B1**

(12) # FASCICULE DE BREVET EUROPEEN

(45) Date de publication de fascicule du brevet: **19.07.95**

(51) Int. Cl.[6]: **C07C 255/23**, C07C 255/19, A61K 31/275

(21) Numéro de dépôt: **93400024.1**

(22) Date de dépôt: **07.01.93**

Le dossier contient des informations techniques présentées postérieurement au dépôt de la demande et ne figurant pas dans le présent fascicule.

(54) **Dérivés 2-cyano 3-hydroxy énamides, leur procédé de préparation, leur application comme médicaments, les compositions pharmaceutiques les renfermant et les intermédiaires obtenus.**

(30) Priorité: **08.01.92 GB 9200275**

(43) Date de publication de la demande:
**14.07.93 Bulletin 93/28**

(45) Mention de la délivrance du brevet:
**19.07.95 Bulletin 95/29**

(84) Etats contractants désignés:
**AT BE CH DE DK ES FR GB GR IE IT LI LU NL PT SE**

(56) Documents cités:
**EP-A- 0 257 882** **EP-A- 0 484 223**
**EP-A- 0 484 223** **WO-A-91/17748**
**DE-A- 2 555 789** **US-A- 4 346 097**

(73) Titulaire: **ROUSSEL-UCLAF**
**35, Boulevard des Invalides**
**F-75007 Paris (FR)**

(72) Inventeur: **Bartlett, Robert R.**
**Schmittweg 23**
**D-6100 Darmstadt 12 (DE)**
Inventeur: **Kay, David Paul**
**4, Church Path**
**Swindon, Wiltshire (GB)**
Inventeur: **Kuo, Elizabeth Anne**
**8, Bullfinch Close**
**Dorcan,**
**Swindon,**
**Wiltshire (GB)**
Inventeur: **Schleyerbach, Rudolf**
**Finkenweg 10**
**D-6238 Hofheim (DE)**
Inventeur: **Schwab ,Wilfried**
**Auf den Erlen 1D**
**D-6200 Wiesbaden-Auringen (DE)**

(74) Mandataire: **Jacobi, Markus Alexander et al**
**Roussel Uclaf**
**111, Route de Noisy**
**B.P. 9**
**F-93230 Romainville (FR)**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).



Rank Xerox (UK) Business Services
(3.10/3.09/3.3.3)

## Description

La présente invention concerne de nouveaux 2-cyano-3-hydroxy-ènamides, leurs formes tautomères et leurs sels, ainsi que le procédé de préparation, l'application à titre de médicaments de ces nouveaux produits et les compositions les renfermant. Dans DE-A 2555789, EP-A 484223 et EP-A 257882, des produits pharmaceutiques ayant une structure cyano-hydroxyalkylamides ont été décrits.

L'invention a pour objet de nouveaux 2-cyano-3-oxo-ènamides répondant à la formule générale (I) :

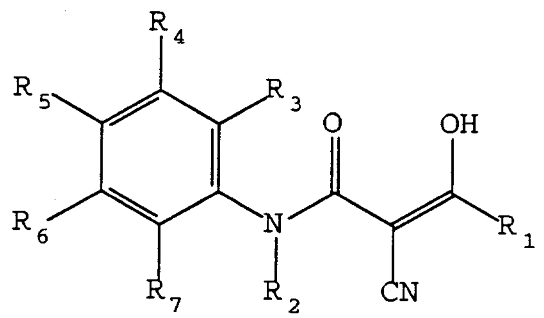

(I)

dans laquelle

- $R_1$ représente un groupement de formule:

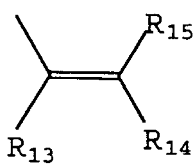 , 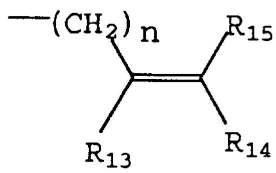 , 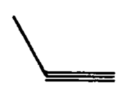

  dans lequel $R_{13}$, $R_{14}$ ou $R_{15}$ représentent un atome d'hydrogène, un atome d'halogène, un radical alkyle renfermant de 1 à 3 atomes de carbone, n représente un nombre entier égal à 1, 2 ou 3,
- $R_2$ représente un atome d'hydrogène, un radical alkyle renfermant de 1 à 3 atomes de carbone,
- $R_3$, $R_4$, $R_5$, $R_6$ et $R_7$, identiques ou différents, représentent un atome d'hydrogène, un atome d'halogène, un groupement nitro ou cyano, un radical alkyle, linéaire ou ramifié, renfermant de 1 à 6 atomes de carbone,

radical cycloalkyle renfermant de 3 à 6 atomes de carbone, un un radical alkoxy, linéaire ou ramifié, renfermant de 1 à 6 atomes de carbone, un radical alkylthio renfermant de 1 à 6 atomes de carbone, un radical $-(CH_2)_m-CF_3$, $-O-(CH_2)_m-CF_3$, $-S-(CH_2)_m-CF_3$ dans lesquels m représente un nombre entier compris entre 0 et 3,
un radical $-CF_2-Hal$, $-OCF_2-Hal$,

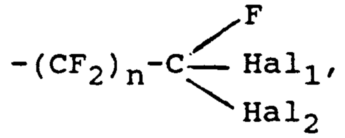

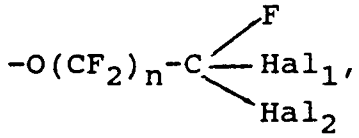 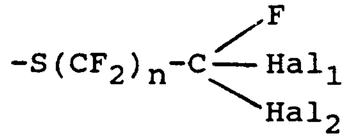 ou 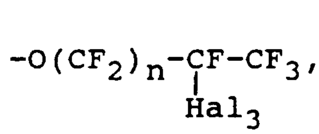

dans lequel n représente un nombre entier compris entre 1 et 3, Hal, $Hal_1$, $Hal_2$ et $Hal_3$, identiques ou différents, représentent un atome d'halogène,
ou $R_3$, $R_4$, $R_5$, $R_6$ et $R_7$, identiques ou différents, représentent un groupement de formule :

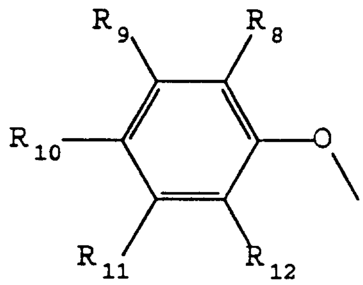

dans lequel $R_8$, $R_9$, $R_{10}$, $R_{11}$ et $R_{12}$, identiques ou différents, représentent l'une quelconque des valeurs indiquées ci-dessus pour $R_3$, $R_4$, $R_5$, $R_6$ et $R_7$ ou $R_4$ et $R_5$ forment ensemble un groupement $-O-CH_2-O$ et $R_3$, $R_6$ et $R_7$ ont la signification indiquée ci-dessus, leurs formes tautomères, ainsi que leurs sels d'addition avec les bases minérales ou organiques.

Dans la formule générale (I) et dans ce qui suit :

- par radical alkyle renfermant de 1 à 3 atomes de carbone, on entend un radical méthyle, éthyle, propyle, isopropyle;
- par radical alkyle renfermant de 1 à 6 atomes de carbone, on entend de préférence un radical méthyle, éthyle, propyle, isopropyle, butyle linéaire ou ramifié, pentyle linéaire ou ramifié, hexyle linéaire ou ramifié,
- par radical alkylcarbonyle renfermant de 2 à 6 atomes de carbone, on entend par exemple acétyle, éthylcarbonyle, propylcarbonyle, butylcarbonyle, isobutylcarbonyle ou isopentylcarbonyle;
- par radical cycloalkyle renfermant de 3 à 6 atomes de carbone, on entend un radical cyclopropyle, cyclobutyle, cyclopentyle ou cyclohexeyl;
- par radical alkoxy comportant de 1 à 6 atomes de carbone, on entend par exemple, un radical méthoxy, éthoxy, propoxy, isopropoxy, butoxy linéaire ou ramifié, pentyloxy linéaire ou ramifié, hexyloxy linéaire ou ramifié,
- par radical alkylthio comportant de 1 à 6 atomes de carbone, on entend par exemple, un radical méthylthio, éthylthio, propylthio, isopropylthio, butylthio linéaire ou ramifié, pentylthio linéaire ou ramifié, hexylthio linéaire ou ramifié,
- par atome d'halogène, on entend de préférence un atome de fluor, de chlore, de brome ou d'iode et de préference un atome de fluor, de chlore ou de brome.

Les sels d'addition avec les bases minérales ou organiques peuvent être, par exemple, les sels formés avec les bases minérales tels que les sels de sodium, de potassium, de lithium, de calcium, de magnésium ou d'ammonium. On peut citer, parmi les bases organiques, la méthylamine, la propylamine, la triméthylamine, la diéthylamine, la triéthylamine, la N,N-diméthyléthanolamine, le tris(hydroxyméthyl) aminométhane, l'éthanolamine, la pyridine, la picoline, la dicyclohexylamine, la morpholine, la benzylamine, la procaïne, la lysine, l'arginine, l'histidine, la N-méthylglucamine.

Parmi les produits, objet de l'invention, on peut citer notamment les dérivés répondant à la formule (I) ci-dessus, ainsi que leurs sels, caractérisés en ce que dans ladite formule (I), $R_3$, $R_4$, $R_5$, $R_6$ et $R_7$, identiques ou différents, représentent un atome d'hydrogène, un atome de fluor, de chlore, de brome ou d'iode, un radical méthyle, éthyle, terbutyle, méthoxy, méthylthio, trifluorométhyle, trifluorométhoxy, trifluorométhylthio, pentafluoroéthyle, bromodifluorométhoxy, cyano, nitro, un groupement phénoxy, 4-chlorophénoxy ou $R_4$ et $R_5$ forment ensemble un groupement $-O-CH_2-O-$, $R_2$ représente un atome d'hydrogène ou un radical méthyle, $R_1$, $R_3$, $R_6$ et $R_7$ ont la signification déjà indiquée, ainsi que leurs sels d'addition avec les bases minérales ou organiques.

Parmi ces derniers, on peut citer plus particulièrement les dérivés répondant à la formule (I) ci-dessus, caractérisés en ce que dans ladite formule (I), $R_1$ représente un groupement:

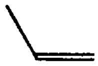, 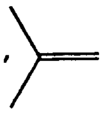, 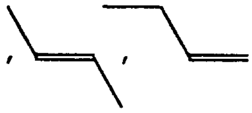, 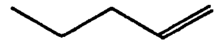, 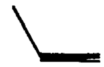

$R_2$ représente un atome d'hydrogène ou un radical méthyle, $R_3$, $R_4$, $R_5$, $R_6$ et $R_7$ identiques ou différents, représentent un atome d'hydrogène, un atome de fluor, de chlore ou de brome, un radical méthyle, trifluorométhyle, trifluorométhoxy ou nitro, cyano ou un groupement phénoxy, ainsi que leurs sels d'addition avec les bases minérales ou organiques.

Parmi ces derniers on retient tout particulièrement les dérivés de formule (I) dont les noms suivent :
2-cyano-3-hydroxy-4-méthyl-N-(4-trifluorométhylphény)-penta-2,4-diénamide;
2-cyano-3-hydroxy-N-(4-trifluorométhylphényl)-hexa-2,5-diénamide;
2-cyano-3-hydroxy-4-méthyl-N-(4-chloro-3-trifluorométhylphényl)-penta-2,4-diénamide;
2-cyano-3-hydroxy-4-méthyl-N-(4-trifluorométhoxyphényl)-penta-2,4-diénamide;
2-cyano-3-hydroxy-4-méthyl-N-(4-bromophényl)-penta-2,4-diénamide;
2-cyano-3-hydroxy-N-(4-chloro-3-trifluorométhylphényl)-hexa-2,5-diénamide;
2-cyano-3-hydroxy-N-(3-méthyl-4-trifluorométhylphényl)-hepta-2,6-diénamide;
2-cyano-3-hydroxy-N-(4-trifluorométhylphényl)-hepta-2,6-diénamide;
ainsi que leurs sels d'addition avec les bases minérales ou organiques.

L'invention a également pour objet un procédé de préparation des nouveaux 2-cyano-3-oxo-ènamides tels que définis par la formule (I) ci-dessus, ainsi que de leurs sels, caractérisé en ce que l'on fait réagir un produit de formule (II) :

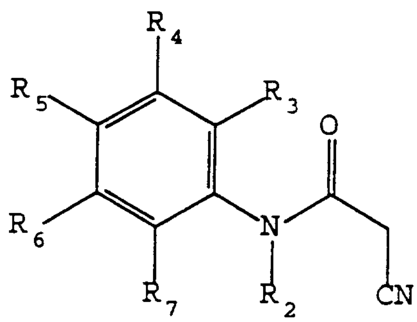

(II)

dans laquelle $R_2$, $R_3$, $R_4$, $R_5$, $R_6$ et $R_7$ ont la signification déjà indiquée, avec successivement de l'hydrure de sodium, si nécessaire en présence d'un catalyseur, puis :

a) soit avec le composé de formule (III):

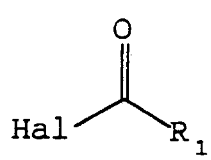

(III)

dans laquelle Hal représente un atome d'halogène et $R_1$ a la signification déjà indiquée,

b) ou avec un composé de formule ($III_A$):

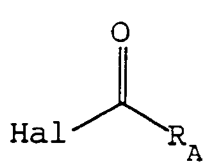

($III_A$)

dans laquelle Hal représente un atome d'halogène et $R_A$ représente un radical $R_1$ dûment protégé, pour

obtenir un produit de formule ($I_A$):

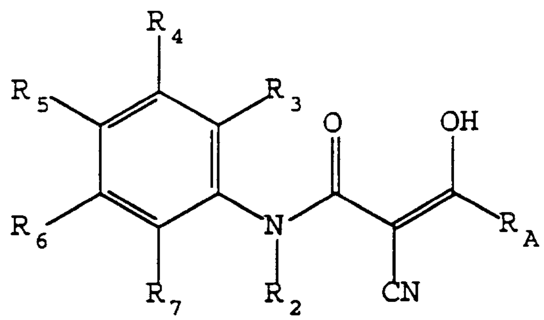

$(I_A)$

dans laquelle $R_A$, $R_2$, $R_3$, $R_4$, $R_5$, $R_6$ et $R_7$ ont la signification déjà indiquée, puis, le cas échéant, déprotège le produit de formule ($I_A$) ainsi obtenu, pour obtenir un produit de formule (I) correspondant, que l'on isole et si désiré salifie.

L'invention a également pour objet un procédé de préparation des produits de formule (I) définie ci-dessus dans laquelle $R_1$ représente un groupe

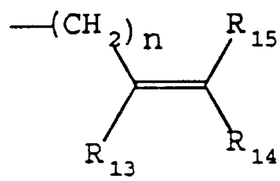

dans lequel $R_{13}$, $R_{14}$ et $R_{15}$ ont la signification déjà indiquée et n a la valeur 2 ou 3, caractérisé en ce que l'on fait réagir un composé de formule (V):

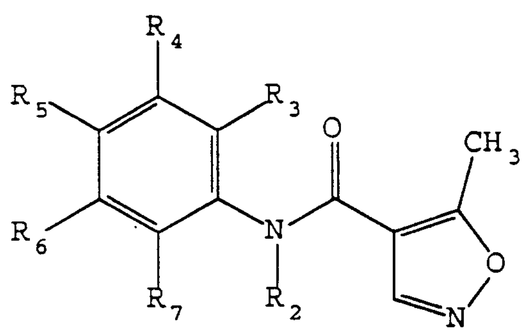

(V)

dans laquelle $R_2$, $R_3$, $R_4$, $R_5$, $R_6$ et $R_7$ ont la signification indiquée ci-dessus avec un composé de formule (VI)

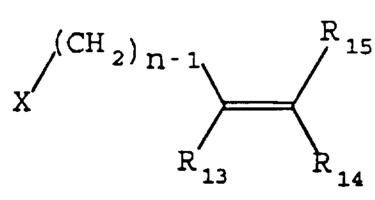

(VI)

dans laquelle X représente un groupement facilement éliminable de préférence l'iode et n, $R_{13}$, $R_{14}$ et $R_{15}$ ont la définition indiquée ci-dessus, en présence d'une base forte pour obtenir le produit de formule (I)

correspondant que l'on isole et si désiré salifie.

Dans des conditions préférentielles de mise en oeuvre de l'invention, le procédé de préparation ci-dessus décrit est caractérisé en ce que :

- la réaction du produit de formule (II) avec l'hydrure de sodium est effectuée au sein d'un solvant organique tel que le tétrahydrofuranne, le cas échéant, en présence d'un catalyseur tel que l'imidazole,
- la réaction avec le composé de formule (III) ou ($III_A$) est effectuée au sein d'un solvant organique tel que le tétrahydrofuranne à basse température, soit aux environs de 0 ° C, soit dans certains cas à une température comprise entre -50 ° et -80 ° C,
- le dérivé fonctionnel de l'acide de formule (III) peut être par exemple le fluorure de propynoyl, préparé in situ par action d'acide propiolique sur du fluorure de benzoyle,
- le radical $R_1$ peut être protégé par un groupement arylseleno tel qu'un groupement phénylseleno,
- la déprotection peut être effectuée par oxydation au moyen d'un péroxyde tel que le péroxyde d'hydrogène en l'absence de solvant ou bien au sein d'un solvant ou d'un mélange de solvants organiques tel que le mélange méthanol-dichlorométhane,
- la réaction du composé de formule (V) avec le composé de formule (VI) ou (VI') est effectuée dans un solvant organique tel que le tétrahydrofuranne, à basse température,
- la base forte utilisée peut être le butyllithium.

Les produits de formule (I) présentent un caractère acide. On peut avantageusement préparer les sels d'addition des produits de formule (I) en faisant réagir, en proportions sensiblement stoéchiométriques, une base minérale ou organique avec lesdits produits de formule (I). Les sels peuvent être préparés sans isoler les acides correspondants.

Les produits, objet de la présente invention, possèdent de très intéressantes propriétés pharmacologiques. On note en particulier une remarquable activité anti-inflammatoire. Ils inhibent d'une part les phénomènes inflammatoires provoqués par des agents irritants, et d'autre part les réactions d'hypersensibilité retardée, en empêchant l'activation des cellules immunitaires par un antigène spécifique.

Ces propriétés sont illustrées plus loin dans la partie expérimentale.

Ces propriétés justifient l'utilisation des nouveaux 2-cyano-3-oxo-ènamides répondant à la formule (I), ainsi que de leurs sels d'addition avec les bases pharmaceutiquement acceptables, à titre de médicaments.

La présente invention a aussi également pour objet l'application à titre de médicaments des nouveaux 2-cyano-3-oxo-ènamides tels que définis par la formule générale (I), ainsi que de leurs sels d'addition avec les bases minérales ou organiques pharmaceutiquement acceptables.

Parmi les médicaments, objet de l'invention, on retient notamment les médicaments, caractérisés en ce qu'ils sont constitués par les nouveaux 2-cyano-3-oxo-ènamides répondant à la formule (I) dans laquelle $R_3$, $R_4$, $R_5$, $R_6$ et $R_7$, identiques ou différents, représentent un atome d'hydrogène, un atome de fluor, de chlore, de brome ou d'iode, un radical méthyle, éthyle, terbutyle, méthoxy, méthylthio, trifluorométhyle, trifluorométhoxy, trifluorométhylthio, pentafluoroéthyle, bromodifluorométhoxy, cyano, nitro, un groupement phénoxy, 4-chlorophénoxy ou $R_4$ et $R_5$ forment ensemble un groupement $-O-CH_2-O-$, $R_2$ représente un atome d'hydrogène ou un radical méthyle, $R_1$, $R_3$, $R_6$ et $R_7$ ont la signification déjà indiquée, ainsi que leurs sels d'addition avec les bases minérales ou organiques pharmaceutiquement acceptables.

Parmi les médicaments, objet de l'invention, on retient plus particulièrement ceux répondant à la formule (I) ci-dessus dans laquelle $R_1$ représente un groupement:

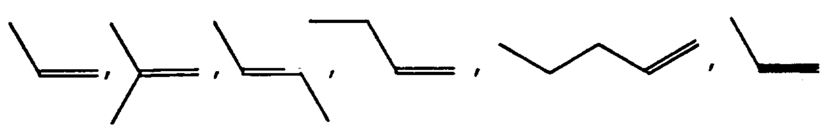

$R_2$ représente un atome d'hydrogène ou un radical méthyle, $R_3$, $R_4$, $R_5$, $R_6$ et $R_7$ identiques ou différents, représentent un atome d'hydrogène, un atome de fluor, de chlore ou de brome, un radical méthyle, trifluorométhyle, trifluorométhoxy, nitro, cyano ou un groupement phénoxy, ainsi que leurs sels d'addition avec les bases minérales ou organiques pharmaceutiquement acceptables.

Parmi les médicaments préférés de l'invention, on retient tout particulièrement :

2-cyano-3-hydroxy-4-méthyl-N-(4-trifluorométhylphényl)-penta-2,4-diénamide;
2-cyano-3-hydroxy-N-(4-trifluorométhylphényl)-hexa-2,5-diénamide;
2-cyano-3-hydroxy-4-méthyl-N-(4-chloro-3-trifluorométhylphényl)-penta-2,4-diénamide;
2-cyano-3-hydroxy-4-méthyl-N-(4-trifluorométhoxyphényl)-penta-2,4-diénamide;

2-cyano-3-hydroxy-4-méthyl-N-(4-bromophényl)-penta-2,4-diénamide;
2-cyano-3-hydroxy-N-(4-chloro-3-trifluorométhylphényl)-hexa-2,5-diénamide;
2-cyano-3-hydroxy-N-(3-méthyl-4-trifluorométhylphényl)-hepta-2,6-diénamide;
2-cyano-3-hydroxy-N-(4-trifluorométhylphényl)-hepta-2,6-diénamide;
ainsi que leurs sels d'addition avec les bases minérales ou organiques pharmaceutiquement acceptables.

Ces médicaments trouvent, par exemple, leur emploi dans le traitement de l'arthrite rhumatoïde et des maladies inflammatoires chroniques d'origine immune ou non (greffes, transplantations d'organes, uvéites, cancers ou autres maladies liées à l'immunologie.

La dose usuelle variable selon le produit utilisé, le sujet traité et l'affection en cause peut être par exemple, de 0,1 mg à 200 mg par jour, par voie orale.

L'invention a également pour objet les compositions pharmaceutiques qui renferment au moins un dérivé précité ou l'un de ses sels d'addition avec les bases pharmaceutiquement acceptables à titre de principe actif.

A titre de médicaments, les dérivés répondant à la formule (I) et leurs sels d'addition avec les bases pharmaceutiquement acceptables peuvent être incorporés dans des compositions pharmaceutiques destinées à la voie digestive ou parentérale.

Ces compositions pharmaceutiques peuvent être, par exemple, solides ou liquides et se présenter sous les formes pharmaceutiques couramment utilisées en médecine humaine, comme par exemple, les comprimés simples ou dragéifiés, les gélules, les capsules, les granulés, les suppositoires, les préparations injectables ; elles sont préparées selon les méthodes usuelles. Le ou les principes actifs peuvent être incorporés à des excipients habituellement employés dans ces compositions pharmaceutiques, tels que le talc, la gomme arabique, le lactose, l'amidon, le stéarate de magnésium, le beurre de cacao, les véhicules aqueux ou non, les corps gras d'origine animale ou végétale, les dérivés paraffiniques, les glycols, les divers agents mouillants, dispersants ou émulsifiants, les conservateurs.

Certains produits de formule (II) sont décrits dans la demande de brevet européen n° 91402890.7 déposée par la demanderesse le 29 octobre 1991. Certains produits de formule (II) sont nouveaux:

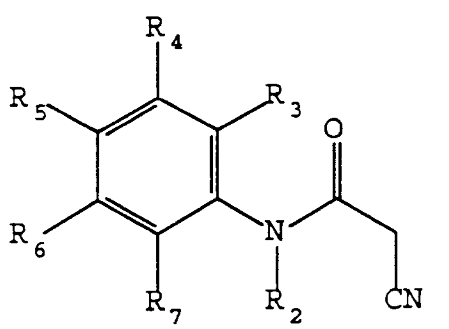

(II)

dans laquelle $R_2$ a la signification indiquée précédemment, l'un des substituants $R_3$, $R_4$, $R_5$, $R_6$ et $R_7$ représente un radical cycloalkyle renfermant de 3 à 6 atomes de carbone ou un groupement

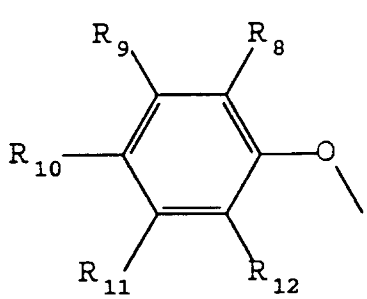

tel que défini ci-dessus, les autres substituants ayant la signification indiquée précédemment.

Parmi les produits de formule (II), on peut citer plus particulièrement :
le [4-(4'-chlorophénoxy)phényl]-cyanoacétamide ; et
le [4-(4'-trifluorométhylphénoxy)phényl]-cyanoacétamide

Les produits de formule (II) peuvent être préparés comme indiqué dans la demande de brevet

européen N° 91402890.7 déposée par la demanderesse le 29 Octobre 1991, au départ de produits de formule (IV) :

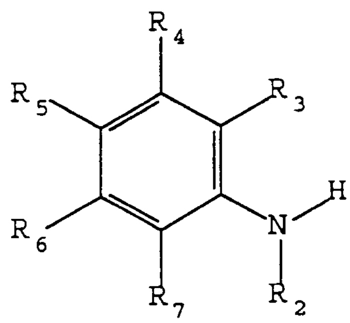

(IV)

dans laquelle $R_2$, $R_3$, $R_4$, $R_5$, $R_6$ et $R_7$ ont la signification déja indiquée, selon un mode opératoire analogue à celui décrit par A. Nohara, T. Ishiguro et al dans J. Med. Chem. (1985) 28 (5), 559-566 selon le schéma suivant :

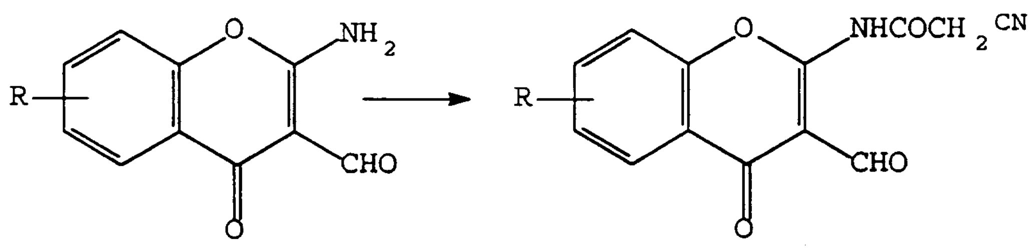

Les produits de formule (IV) utilisés au départ du procédé sont en général des produits connus ou peuvent être préparés par diazotation (réaction d'un sel de diazonium avec un sel de métal alcalin ou de cuivre, approprié (chlorure de cuivre, iodure de potassium, cyanure de sodium), puis réduction des nitroanilines correspondantes selon un procédé connu de l'homme de métier.

Les nitroanilines utilisées peuvent être préparées comme indiqué par exemple dans TP. Sura et al. Synthetic communications (1988) 18 (16-17) 2161-5.

Certaines anilines de formule (IV) peuvent être préparées selon le brevet européen EP. 206951 ou par réduction de nitrobenzènes correspondants qui sont connus en général.

Certains nitrobenzènes sont nouveaux et peuvent être préparés comme indiqué ci-après dans les exemples.

Les composés de formule (V) utilisés dans le procédé ci-dessus sont en général des produits connus ou peuvent être préparés selon le procédé décrit dans la demande de brevet n° WO.91/17748.

Il va être donné maintenant à titre non limitatif, des exemples de mise en oeuvre de l'invention.

**EXEMPLE 1: 2-Cyano-3-hydroxy 4-méthyl-N-(4trifluorométhylphényl)pent-2,4-diénamide (Méthode F).**

On a préparé le produit attendu à partir des produits de départ appropriés en utilisant la méthode décrite à l'exemple 8.

**EXEMPLE 2: 2-Cyano-3-hydroxy-N-(4-trifluorométhylphényl)pent-2,4-diénamide** (methode C).

Stade A : 2-cyano 3-hydroxy 5-phénylseleno N-(4-trifluorométhylphényl) penta-2,4-diénamide.

On mélange sous azote 7 g de 4'-trifluorométhylcyano acétanilide dans 200 $cm^3$ de tétrahydrofuranne en ajoutant 0,02 g d'imidazole et 2,3 g d'hydrure de sodium. On agite 2 heures à température ambiante, refroidit à -78°C et ajoute 9,11 g de chlorure de 3-(phénylseleno)propionyle préparé comme indiqué dans J. Med. Chem. (1988) 31 1190-6. On agite à -78°C pendant 90 minutes, verse dans un mélange acide chlorhydrique/glace et filtre. On dissout le résidu solide dans le chlorure de méthylène, lave à l'eau, sèche

et élimine le solvant sous pression réduite. Après trituration dans l'éther éthylique, on obtient 13,4 g de produit attendu.

Stade B : 2-Cyano-3-hydroxy N-(4-trifluorométhylphényl)pent-2,4-diénamide (methode C).

On refroidit à 0 °C, 8 g de produit obtenu au stade A dans 200 $cm^3$ de chlorure de méthylène, ajoute 4 $cm^3$ d'eau oxygénée à 30% et agite 30 minutes la suspension de selenoxyde obtenue. On dilue avec 40 $cm^3$ de méthanol et 200 $cm^3$ de chlorure de méthylène, agite 1 heure à température ambiante et filtre sur silice. On élimine le solvant sous pression réduite, reprend le résidu dans l'éther éthylique et obtient 2,8 g de produit attendu.

**EXEMPLE 3: 2-Cyano-3-hydroxy 4-méthyl N-(4-bromo-3-méthylphényl)pent-2,4-diénamide** (methode A).

On agite sous azote 6,3 g de 4'-bromo 3'-méthylcyano acétanilide en solution dans 200 $cm^3$ de tétrahydrofuranne en ajoutant 0,02 g d'imidazole et 1,85 g d'hydrure de sodium. On agite la suspension pendant 1 heure à température ambiante, refroidit à -78 °C, ajoute 2,95 $cm^3$ de chlorure de métacryloyle fraîchement distillé, et agite à -20 °C pendant 90 minutes. On verse dans un mélange acide chlorhydrique/glace et filtre. On dissout le résidu solide dans du chlorure de méthylène, lave à l'eau, sèche et élimine le solvant sous pression réduite. On obtient 8 g de produit attendu.

**EXAMPLE 4: 2-Cyano-3-hydroxy N-(4-trifluoromethylphenyl)pent-2,4-diènamide** (méthode E).

On mélange sous azote 5 g de 4'-trifluorométhylcyano acétanilide dans 150 $cm^3$ de tétrahydrofuranne et ajoute 2 g d'hydrure de sodium. On agite 1 heure à température ambiante et refroidit à -70 °C. Au ballon contenant le milieu réactionnel, on adapte un condenseur acétone/glace relié à un appareil à distillation chargé de 3,01 $cm^3$ d'acide propiolique et 15 g de fluorure de benzoyle tel que décrit dans JACS (1974) 96 (18) 5855-9. On chauffe à 150 °C et le fluorure de propionyle libéré condense dans la solution du carbanion. On agite à - 70 °C pendant 1 heure, verse dans un mélange acide chlorhydrique/glace, extrait à l'acétate d'éthyle, sèche et élimine le solvant sous pression réduite. Après trituration dans l'éther éthylique et chromatographie des liqueurs mères sur silice (éluant : chlorure de méthylène), on obtient 2,49 g de produit attendu.

**EXAMPLE 5: (E)-2-Cyano-3-hydroxy N-(4-trifluorométhylphényl)-hex-2,4-diénamide** (méthode B).

On mélange sous azote 6 g de 4'-trifluorométhylcyano acétanilide dans 200 $cm^3$ de tétrahydrofuranne en ajoutant 0,02 g d'imidazole et 1,95 g d'hydrure de sodium. On agite 2 heures à température ambiante, refroidit à -78 °C et ajoute 3,06 g de chlorure de crotonoyle fraîchement distillé. On agite à -78 °C pendant 2 heures, verse dans un mélange acide chlorhydrique/glace et filtre. On dissout le résidu solide dans le chlorure de méthylène, lave à l'eau, sèche et élimine le solvant sous pression réduite. On obtient 7,65 g de produit attendu.

**EXEMPLE 6: 2-Cyano-3-hydroxy N-(4-trifluorométhylphényl)hex-2,5-diénamide** (méthode D).

On mélange sous azote 6 g de 4'-trifluorométhylcyano acétanilide dans 200 $cm^3$ de tétrahydrofuranne et ajoute 1,95 g d'hydrure de sodium. On agite 30 minutes à température ambiante, refroidit à -50 °C et ajoute 3,3 g de chlorure de 3-buténoyle préparé comme indiqué dans J. Chem. Soc. (1948) 661. On agite à -50 °C pendant 2 heures, verse dans un mélange acide chlorhydrique/glace et filtre. Après chromatographie du résidu sur silice (éluant : chlorure de méthylène), on obtient 2,4 g de produit attendu.

**EXEMPLE 7: 2-Cyano-3-hydroxy 4-méthyl N-(4-chloro-3-trifluorométhylphényl)pent-2,4-diénamide.**

On a préparé le produit attendu à partir des produits de départ appropriés en utilisant la méthode décrite à l'exemple 8.

**EXEMPLE 8: 2-Cyano-3-hyroxy 4-méthyl N-(4-trifluorométhoxyphényl)pent-2,4-diénamide** (méthode F).

On agite sous azote 0,5 g de 4'trifluorométhoxycyano acétanilide en solution dans 22 $cm^3$ de tétrahydrofuranne en ajoutant une quantité catalytique d'imidazole et 0,15 g d'hydrure de sodium. On agite la suspension pendant 10 minutes à température ambiante, refroidit à -78°C, ajoute 0,24 $cm^3$ de chlorure de métacrynoyle fraîchement distillé, et agite pendant 30 minutes. On ajoute 0,3 $cm^3$ d'acide acétique glacial, agite de nouveau 30 minutes. On verse dans de l'acide chlorhydrique glacé, filtre, rince avec 3 fois 5 $cm^3$ d'eau et 5 $cm^3$ d'éther et obtient après séchage 575 mg de produit attendu.

**EXAMPLE 9: 2-Cyano-3-hydroxy-4-methyl N-(4-(4'chlorophenoxy)phenyl]-penta-2,4-dienamide.**

On a préparé le produit attendu à partir des produits de départ appropriés sans utiliser d'imidazole en opérant selon la méthode décrite à l'exemple 8.

**EXEMPLE 10: 2-Cyano-3-hydroxy 4-méthyl N-[4-bromophényl]pent-2,4-diénamide. (méthode F).**

On a préparé le produit attendu à partir des produits de départ appropriés en utilisant la méthode décrite à l'exemple 8.

**EXAMPLE 11: 2-Cyano-3-hydroxy-4-méthyl-N-[4-(4'-trifluorométhylphénoxy)phényl]-penta-2,4-diénamide**

On a préparé le produit attendu à partir des produits de départ appropriés en utilisant la méthode décrite à l'exemple 8.

**EXEMPLE 12: 2-Cyano-3-hydroxy-N-(4-trifluorométhylphényl)hepta-2,6-diénamide (Méthode G).**

On dissout 6,75 g de 5-méthyl 4-[N-(4-trifluorométhyl) phényl] carbamoyl-isoxazole dans 500 $cm^3$ de tétrahydrofuranne sous atmosphère inerte. On refroidit à -78°C, ajoute 32 $cm^3$ d'une solution 2,5N de butyllithium dans l'hexane, agite 90 minutes, ajoute 10,8 $cm^3$ d'iodure d'allyle, poursuit l'agitation pendant 2 heures; ajoute 20 $cm^3$ d'eau et laisse revenir à 0°C. On ajoute alors, 500 $cm^3$ d'acétate d'éthyle et 200 $cm^3$ d'acide chlorhydrique (1N), sépare la phase organique, la lave à l'eau, sèche et évapore le solvant. Après cristallisation, du mélange acétone/eau/acide chlorhydrique, on obtient 6,35 g de produit attendu. F = 145°C.

**EXAMPLE 13 : 2-Cyano-3-hydroxy-N-(4-chloro-3-méthylphényl)-hepta-2,6-diénamide.**

On a obtenu 7,5 g de produit attendu à partir du 5-méthyl-4-(N-(4-chloro-3-méthyl)-phényl)carbamoyl-isoxazole en utilisant la méthode décrite à l'exemple 12 (Méthode G). F = 134°C.

En opérant comme indiqué dans l'exemple 3 (Méthode A) ou 6 (Méthode D) à partir des produits appropriés, on a préparé les produits suivants :

**EXEMPLE 16 : 2-Cyano-3-hydroxy-4-méthyl-N-(4-chlorophényl)-penta-2,4-diénamide (Méthode A).**

**EXEMPLE 17 : 2-Cyano-3-hydroxy-4-méthyl-N-(4-iodophényl)-penta-2,4-diénamide (Méthode A).**

**EXEMPLE 18 : 2-Cyano-3-hydroxy-4-méthyl-N-(4-fluorophényl)-penta-2,4-diénamide (Méthode A).**

**EXEMPLE 19 : 2-Cyano-3-hydroxy-4-méthyl-N-(3-méthyl-4-trifluorométhylphényl)-penta-2,4-diénamide (Méthode A).**

**EXEMPLE 20 : 2-Cyano-3-hydroxy-4-méthyl-N-(4-cyanophényl)-penta-2,4-diénrnide (Méthode A).**

**EXEMPLE 21 : 2-Cyano-3-hydroxy-4-méthyl-N-(4-nitrophényl)-penta-2,4-diénamide (Méthode A).**

**EXEMPLE 22 : 2-Cyano-3-hydroxy-N-(4-trifluorométhoxyphényl)-hexa-2,5-diénamide (Méthode D).**

**EXEMPLE 23 : 2-Cyano-3-hydroxy-N-(4-trifluorométhylthiophényl)-hexa-2,5-diénamide (Méthode D).**

**EXEMPLE 24 : 2-Cyano-3-hydroxy-N-(4-chloro-3-trifluorométhylphényl)-hexa-2,5-diénamide (Méthode D).**

**EXEMPLE 25 : 2-Cyano-3-hydroxy-N-(4-fluorophényl)-hexa-2,5-diénamide (Méthode D).**

**EXEMPLE 26 : 2-Cyano-3-hydroxy-N-(3,4-difluorophényl)-hexa-2,5-diénamide (Méthode D).**

**EXEMPLE 27 : 2-Cyano-3-hydroxy-N-(2,4-difluorophényl)-hexa-2,5-diénamide (Méthode D).**

**EXEMPLE 28 : 2-Cyano-3-hydroxy-4-méthyl-N-(3-trifluorométhylphényl)-penta-2,4-diénamide (Méthode A).**

**EXEMPLE 29 : 2-Cyano-3-hydroxy-4-méthyl-N-(3,4-difluorophényl)-penta-2,4-diénamide (Méthode A).**

**EXEMPLE 30 : 2-Cyano-3-hydroxy-4-méthyl-N-(3-chloro-4-fluorophényl)-penta-2,4-diénamide (Méthode A).**

**EXEMPLE 31 : 2-Cyano-3-hydroxy-4-méthyl-N-(3,4-dichlorophényl)-penta-2,4-diénamide (Méthode A).**

**EXEMPLE 32 : 2-Cyano-3-hydroxy-N-(4-chlorophényl)-hepta-2,6-diénamide.**

On a obtenu 4,3 g du produit attendu à partir du 5-méthyl-4-(N-(4-chlorophényl)carbamoyl-isoxazole en opérant selon la méthode décrite à l'exemple 12 (Méthode G). F = 138 °C.

**EXEMPLE 33 : 2-Cyano-3-hydroxy-N-(3-méthyl-4-trifluorométhylphényl)-hepta-2,6-diénamide.**

On a obtenu 4,29 g de produit attendu à partir du 5-méthyl-4-(N-(4-trifluorométhyl-3-méthyl)-phényl)-carbamoyl-isoxazole en opérant selon la méthode décrite à l'exemple 12 (Méthode G). F = 133 °C.

Les analyses spectrométriques, les rendements, les points de fusion et les résultats de la microanalyse de ces composés sont donnés dans le tableau I.

TABLEAU I

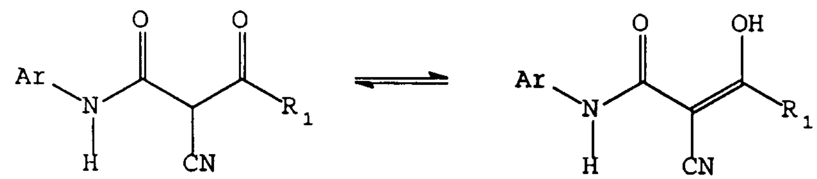

| Ex | Ar | $R_1$ | Rendt % | F°C | IR $cm^{-1}$ | $^1H$ RMN δ | Formule P.M. | Analyse % Calc. | Trouvé |
|---|---|---|---|---|---|---|---|---|---|
| 1 | 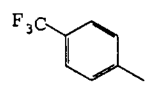 | 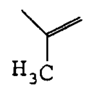 | 69 Méthode F | 191-193 | 3275(NH), 2220(CN), 1630, 1598, 1541, 1320, 1106, 1062, 832. | $CDCl_3$ 17.09(1H,s); 7.44 (2H,d); 7.21 (2H,d); 3.34 (3H,s); 2.13 (1H,m); 1.26 (2H,m); 1.05 (2H,m). | $C_{14}H_{11}F_3$-$N_2O_2$ 296.25 | C 56.76<br>H 3.74<br>F 19.24<br>N 9.46<br>O 10.80 | 56.85<br>3.82<br>19.17<br>9.47<br>- |
| 2 | 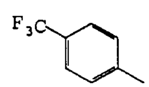 | 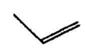 | 54 Méthode C | 206-208 | 3300(NH), 2215(CN), 1632, 1604, 1550, 1530, 1412, 1332, 1321, 1111, 1069, 837. | DMSO-$d_6$ 12.17(1H,s); 7.81 (2H,d); 6.88(1H,dd); 6.20(1H,dd); 5.69(1H,dd). | $C_{13}H_9F_3$-$N_2O_2$ 282.22 | C 55.33<br>H 3.21<br>F 20.20<br>N 9.93<br>O 11.34 | 55.23<br>3.27<br>20.27<br>10.00<br>- |
| 3 | 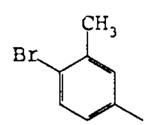 | 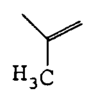 | 100 Méthode A | 143-144 | 3300(NH), 2220(CN), 1608, 1542, 1481, 1357, 1310, 1028, 935. | $CDCl_3$ 15.90(1H,s); 7.73 (1H,s); 7.52 (1H,d); 7.39 (1H,d); 7.23(1H,dd); 6.04 (1H,s); 5.31 (1H,s); 2.41 (3H,s); 2.08 (3H,s). | $C_{14}H_{13}Br$-$N_2O_2$ 321.18 | C 52.36<br>H 4.08<br>Br24.88<br>N 8.72<br>O 9.96 | 52,42<br>4,13<br>24,90<br>8,74 |

TABLEAU I (suite)

| Ex | Ar | $R_1$ | Rendt % | F°C | IR $cm^{-1}$ | $^1H$ RMN δ | Formule P.M. | Analyse % Calc. | Trouvé |
|---|---|---|---|---|---|---|---|---|---|
| 4 | 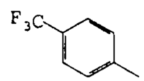 |  | 40 Méthode E | 170 dec | 3290(NH), 2215(CN), 2109, 1622, 1604, 1324, 1259, 1141, 1100, 1064. | DMSO-$d_6$ 11.96(1H,s); 7.74 (2H,d); 7.62 (2H,d); 4.23 (1H,s). | $C_{13}H_7F_3N_2O_2$ 280.21 | C 55.72<br>H 2.52<br>F 20.34<br>N 10.00<br>O 11.42 | 55.41<br>2.63<br>20.35<br>9.99<br>- |
| 5 | 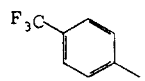 | 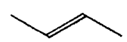 | 99 Méthode B | 210-220 subl. | 3315(NH), 2219(CN), 1650, 1631, 1610, 1565, 1555, 1330, 1160, 1118, 1072, 842. | DMSO-$d_6$ 11.24(1H,s); 9.15(1H,s); 7.84(2H,d); 7.72(2H,d); 6.97(1H,sxt); 6.56(1H,dd); 1.99(3H,dd). | $C_{14}H_{11}F_3N_2O_2$ 296.25 | C 56.76<br>H 3.74<br>F 19.24<br>N 9.46<br>O 10.80 | 56.40<br>3.88<br>18.82<br>9.42<br>- |
| 6 | 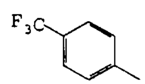 | 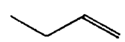 | 31 Méthode D | 166-168 | 3300(NH), 2215(CN), 1629, 1588, 1552, 1381, 1322, 1163, 1120, 1071, 848. | $CDCl_3$ 15.49(1H,s); 7.93 (1H,s); 7.64 (4H,s); 5.90 (1H,m); 5.33 (2H,m); 3.38 (3H,d). | $C_{14}H_{11}F_3N_2O_2$ 296.25 | C 56.76<br>H 3.74<br>F 19.24<br>N 9.46<br>O 10.80 | 56.71<br>3.79<br>19.07<br>9.50<br>- |
| 7 | 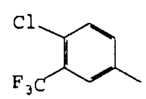 | 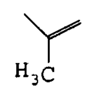 | 63 Méthode F | 159 | 3280(NH), 2205(CN), 1620, 1580, 1550, 1510, 1478, 1450, 1350, 1312, 1300, 1260, 1221, 1170, 1130, 1026, 950,932,893, 878, 820. | DMSO-$d_6$ 12.05(1H,s); 8.28 (1H,m); 7.72 (2H,m); 5.40 (1H,s); 5.33 (1H,s); 1.93 (3H,s). | $C_{14}H_{10}ClF_3$-$N_2O_2$ 330.70 | C 50.85<br>H 3.05<br>Cl10.72<br>F 17.23<br>N 8.47<br>O 9.68 | 50.99<br>3.11<br>-<br>-<br>8.53<br>- |

TABLEAU I (suite)

| Ex | Ar | $R_1$ | Rendt % | F°C | IR $cm^{-1}$ | $^1H$ RMN δ | Formule P.M. | Analyse % Calc. | Trouvé |
|---|---|---|---|---|---|---|---|---|---|
| 8 | 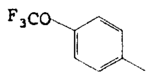 | 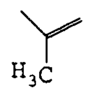 | 90 Méthode F | 156 | 2290, 2210, 1630, 1610, 1550, 1500, 1450, 1410, 1110, 960, 935, 910, 835, 805, 780. | DMSO-$d_6$ 11.38(1H,s); 7.70 (2H,d); 7.36 (2H,d); 5.55 (2H,d); 1.97 (1H,d). | $C_{14}H_{11}F_3$-$N_2O_3$ 312.25 | C 53.85<br>H 3.55<br>F 18.25<br>N 8.97<br>O 15.37 | 54.07<br>3.72<br>-<br>8.96<br>- |
| 9 | 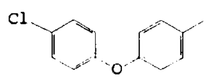 | 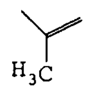 | 58 Méthode F | 115-116 | 3270, 2214, 1602, 1580, 1549, 1502, 1483, 1454, 1416, 1370, 1317, 1298, 1280, 1249, 1229, 1163, 1088, 1008, 843, 823. | $CDCl_3$ 2.09 (3H,s); 5.68 (1H,s); 6.04 (1H,s); 6.95(2H,d+v, J=9.2Hz); 7.31(2H,d+v, J=9.0Hz); 7.46(2H,d+v, J= 9.0Hz); 7.75(1H,br s); 15.97 (1H,s). | $C_{19}H_{15}Cl$-$N_2O_3$ 354.80 | C 64.32<br>H 4.26<br>Cl 9.99<br>N 7.90<br>O 13.53 | 64.41<br>4.33<br>9.98<br>7.90<br>13.38 |
| 10 | 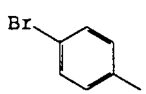 | 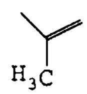 | 65 Méthode F | 148 | 3300, 2220, 1875, 1635, 1480, 1450, 1400, 1370, 1310, 1285, 1240, 1172, 1107, 1065, 1005, 935, 910, 809, 780, 767. | DMSO-$d_6$ 11.08(1H,s); 7.54 (4H,m); 5.62 (2H,d); 1.98 (3H,s). | $C_{13}H_{11}Br$-$N_2O_2$ 307.15 | C 50.84<br>H 3.61<br>Br26.00<br>N 9.12<br>O 10.42 | 51.20<br>3.67<br>-<br>8.79<br>- |

TABLEAU I (suite)

| Ex | Ar | $R_1$ | Rendt % | F°C | IR $cm^{-1}$ | $^1H$ RMN δ | Formule P.M. | Analyse % Calc. | Trouvé |
|---|---|---|---|---|---|---|---|---|---|
| 11 | 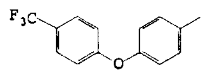 | 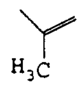 | 69 Méthode F | 155 | 3272, 2214, 1604, 1552, 1501, 1367, 1333, 1316, 1290, 1253, 1233, 1196, 1170, 1156, 1115, 1102, 1060, 1012. 834. | $CDCl_3$ 2.09(3H,s); 5.68(1H,s); 6.05(1H,s); 7.03-7.11(4H,m); 7.51(2H,d, J=8.80Hz); 7.59(2H,d,J=8.60Hz);7.78(1H, s,NH);15.93 (1H,s,OH). | $C_{20}H_{15}F_3$-$N_2O_3$ 388.35 | C 61.86<br>H 3.89<br>N 7.21<br>O 12.36<br>F 14.68 | 61.73<br>3.95<br>7.19<br>12.60<br>14.53 |
| 12 | 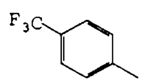 | 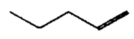 | 82 Méthode G | 145 | 3300(NH), 2220(CN), 1630, 1590, 1555, 1415, 1390, 1315, 1265, 1190, 1165, 1130, 1117, 1070, 1010, 845. | DMSO-$d_6$ 2.25-2.7 (4H,m); 4.98-5.15 (2H, m); 5.88(1H, m); 7.65 and 7.78 (4H, AA'BB'); 11.2 (1H,s). | $C_{15}H_{13}F_3$-$N_2O_2$ 310.28 | C 58.07<br>H 4.22<br>N 9.03<br>O 10.31<br>F 18.37 | |
| 13 | 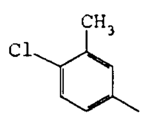 | 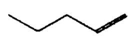 | 66 Méthode G | 134 | 3300(NH), 2215(CN), 1590, 1550, 1480, 1310, 1045, 990, 920, 865, 820, 810. | DMSO-$d_6$ 2.25-2.7 (7H,m); 4.95-5.15 (2H,m); 5.85(1H,m); 7.27-7.55 (3H,m); 10.7(1H,s). | $C_{15}H_{15}Cl$-$N_2O_2$ 290.75 | C 61.97<br>H 5.20<br>N 9.63<br>O 11.01<br>Cl12.19 | |
| 14 | 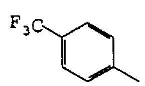 | 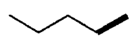 | 52 Méthode G | 172 | 3310(NH), 2220(CN), 1630, 1590, 1550, 1415, 1325, 1160, 1120, 1070, 840. | DMSO-$d_6$ 2.5 and 2.75 (2H each,m); 2.85 (1H,t, J=2.5Hz); 7.64 and 7.78 (4H, AA'BB'); 11.15(1H,s). | $C_{15}H_{11}F_3$-$N_2O_2$ 308.26 | C 58.45<br>H 3.60<br>N 9.09<br>O 10.38<br>F 18.49 | |

TABLEAU I (suite)

| Ex | Ar | $R_1$ | Rendt % | F°C | IR $cm^{-1}$ | $^1H$ RMN δ | Formule P.M. | Analyse % Calc. | Trouvé |
|---|---|---|---|---|---|---|---|---|---|
| 15 | 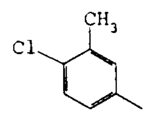 | 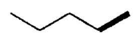 | 44 Méthode G | 147 | 3310(NH), 2220(CN), 1610, 1600, 1590, 1555, 1490, 1315, 825. | DMSO-$d_6$ 2.29 (3H,s); 2.5 and 2.74 (2H each,m); 2.85 (1H,t, J=2.5Hz); 7.28-7.55 (3H,m); 10.8(1H,s). | $C_{15}H_{13}N_2O_2Cl$ | C 62.40<br>H 4.54<br>N 9.70<br>O 11.08<br>Cl12.28 | |
| 16 | 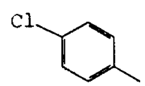 | 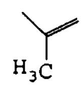 | 85 Méthode A | 149-151 | 3300(NH), 2210(CN), 1890, 1650, 1495, 1460, 1415, 1380, 1325, 1295, 1250, 1180, 1120, 1100, 1095. | DMSO-$d_6$ 11.00(1H,s); 7.60 (2H,m); 7.43 (2H,m); 5.64 (2H,d); 1.98 (3H,s). | $C_{13}H_{13}N_2O_2Cl$<br>262.83 | C 59.44<br>H 4.22<br>N 10.69<br>Cl13.49 | 59.06<br>4.26<br>10.50<br>13.53 |
| 17 | 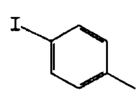 | 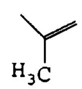 | 68 Méthode A | 154-157 | 3300(NH), 2230(CN), 1620, 1590, 1560, 1530, 1480, 1465, 1390, 1360, 1310, 1285, 1230, 1110, 1060. | DMSO-$d_6$ 11.46(1H,s); 7.61 (2H,d); 7.44 (2H,d); 5.48 (2H,d); 1.94 (3H,s). | $C_{13}H_{11}N_2O_2I$<br>354.15 | C 44.09<br>H 3.13<br>N 7.91<br>I 35.83 | 43.90<br>3.14<br>7.81<br>35.67 |
| 18 | 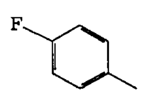 | 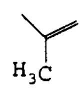 | 70 Méthode A | 111-112 | 3310(NH), 2210(CN), 1880, 1690, 1620, 1550, 1505, 1460, 1415, 1370, 1320, 1270, 1220, 1155, 1100, 1015, 940, 830. | DMSO-$d_6$ 11.05(1H,s); 7.57 (2H,m); 7.19 (2H,m); 5.60 (2H,d); 1.95 (3H,s). | $C_{13}H_{11}N_2O_2F$<br>246.24 | C 63.41<br>H 4.50<br>N 11.38 | 63.50<br>4.80<br>10.96 |

TABLEAU I (suite)

| Ex | Ar | $R_1$ | Rendt % | F°C | IR $cm^{-1}$ | $^1H$ RMN δ | Formule P.M. | Analyse % Calc. | Trouvé |
|---|---|---|---|---|---|---|---|---|---|
| 19 | 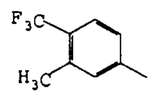 | 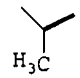 | 84 Méthode A | 147-149 | 3280(NH), 2190(CN), 1635, 1600, 1530, 1440, 1390, 1350, 1300, 1250, 1230, 1140, 1110, 1090, 1030, 820. | DMSO-$d_6$ 11.50(1H,s); 7.62 (3H,s); 5.48 (2H,d): 2.45 (3H,s); 1.97 (3H,s). | $C_{15}H_{13}F_3$-$N_2O_2$ 310.27 | C 58.07 H 4.22 N 9.03 O 10.31 F 18.37 | |
| 20 | 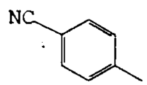 | 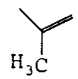 | 77 Méthode A | 175-177 | 3290(NH), 2220(CN), 1915, 1695, 1600, 1580, 1550, 1460, 1415, 1380, 1330, 1310, 1280, 1250, 1190, 1110, 1015. | DMSO-$d_6$ 12.29(1H,s); 7.75 (4H,s); 5.35 (2H,d); 1.92 (3H,s). | $C_{14}H_{11}N_3O_2$ 253.26 | C 66.40 H 4.38 N 16.59 O 12.63 | |
| 21 | 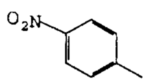 | 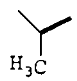 | 85 Méthode A | 208-210 | 3320(NH), 2200(CN), 1635, 1610, 1560, 1500, 1340, 1305, 1245, 1180, 1105, 990, 940, 850. | DMSO-$d_6$ 12.48(1H,s); 8.22 (2H,d); 7.80 (2H,d); 5.37 (2H,d), 1.92 (3H,s). | $C_{13}H_{11}N_3O_4$ 273.24 | C 57.14 H 4.06 N 15.38 | 56.97 4.20 15.20 |
| 22 | 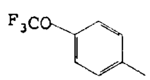 | 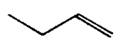 | 70 Méthode D | 148-150 | 3280(NH), 2185(CN), 1596, 1578, 1545, 1495, 1280, 1190, 1130. | $CDCl_3$ 15.60(1H,s); 7.68 (1H,s); 7.52 (2H,m); 7.24 (2H,d); 5.93 (1H,m); 5.32 (2H,m); 3.37 (2H,d). | $C_{14}H_{11}F_3F_3$ 312.26 | C 53.85 H 3.55 N 8.97 F 18.25 | 53.89 3.61 8.99 18.24 |

TABLEAU I (suite)

| Ex | Ar | $R_1$ | Rendt % | F°C | IR $cm^{-1}$ | $^1H$ RMN δ | Formule M.wt | Analyse % Calc. | Trouvé |
|---|---|---|---|---|---|---|---|---|---|
| 23 | 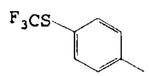 | 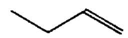 | 73 Méthode D | 145-147 | 3260(NH), 2187(CN), 1590, 1580, 1568, 1530, 1478, 1391, 1368, 1303, 1279, 1170, 1142, 1120, 1110, 1072. | $CDCl_3$ 15.52(1H,s); 7.74 (1H,s); 7.60 (4H,m); 5.94 (1H,m); 5.34 (1H,d, J=15.8Hz); 5.30(1H,d,J=10.2 Hz); 3.38(2H,d,J=6.8 Hz). | $C_{14}H_{11}N_2O_2$-$SF_3$ 328.32 | C 51.22 H 3.38 N 8.53 S 9.76 | 51.46 3.37 8.65 9.66 |
| 24 | 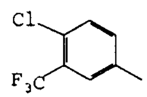 | 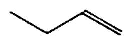 | 61 Méthode D | 160-162 | 3301(NH), 2224(CN), 1629, 1587, 1551, 1487, 1381, 1323, 1177, 1144, 1132. | $CDCl_3$ 15.42(1H,s); 7.90(1H,d,J=2.6 Hz); 7.82 (1H,s); 7.69 (1H,m); 7.51(1H,d,J=8.6 Hz); 5.89 (1H,m); 5.31 (2H,m); 3.38(2H,d,J=6.8 Hz). | $C_{14}H_{10}N_2O_2F$-$_3Cl$ 330.70 | C 50.85 H 3.05 N 8.47 Cl10.72 F 17.24 | 50.79 3.09 8.44 10.71 17.28 |
| 25 | 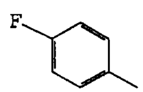 | 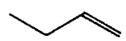 | 55 Méthode D | 129-131 | 3320(NH), 2218(CN), 1595, 1568, 1513, 1418, 1385, 1321, 1267, 1217, 835. | $CDCl_3$ 15.72(1H,s); 7.64 (1H,s); 7.45 (2H,m); 7.10 (2H,m); 5.89 (1H,m); 5.31(2H,dd,J=15.6,11Hz); 3.37(2H,d,J=6.6 Hz). | $C_{13}H_{11}N_2O_2F$ 246.24 | C 63.41 H 4.50 N 11.38 F 7.72 | 63.37 4.57 11.44 7.82 |

TABLEAU I (suite)

| Ex | Ar | $R_1$ | Rendt % | F°C | IR $cm^{-1}$ | $^1H$ RMN δ | Formule M.wt | Analyse % Calc. | Trouvé |
|---|---|---|---|---|---|---|---|---|---|
| 26 | 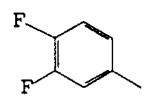 | 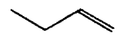 | 48 Méthode D | 135-136 | 3289(NH), 2220(CN), 1599, 1564, 1518, 1439, 1391, 1290, 1252, 1217. | $CDCl_3$ 15.52(1H,s); 7.56 (2H,m); 7.13 (2H,m): 5.89 (1H,m); 5.30 (2H,dd, J=17,9.8Hz); 3.37(2H,d,J= 6.8 Hz). | $C_{13}H_{10}N_2O_2F_2$ 264.23 | C 59.09 H 3.81 N 10.60 F 14.38 | 59.08 3.89 10.62 14.44 |
| 27 | 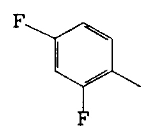 | 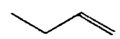 | 38 Méthode D | 68-69 | 3297(NH), 2226(CN), 1597, 1562, 1510, 1385, 1262, 966, 933. | $CDCl_3$ 15.40(1H,s); 7.99 (1H,m); 7.67 (1H,s); 6.93 (2H,m); 5.93 (1H,m); 5.35(2H,dd, J=16.8,11Hz) ;3.38(2H,d, J= 6.8Hz). | $C_{13}H_{10}N_2O_2F_2$ 264.23 | C 59.09 H 3.81 N 10.60 F 14.38 | 59.39 3.97 10.47 13.98 |
| 28 | 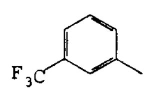 | 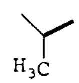 | 76 Méthode A | 131-3 | 3302(NH), 2214(CN), 1640, 1551, 1497, 1370, 1324, 1275, 1171, 1128. | $CDCl_3$ 15.80(1H,s); 7.99 (1H,s); 7.87 (1H,s); 7.69 (1H,m); 7.48 (2H,m); 6.05 (1H,s); 5.69 (1H,m); 2.09 (3H,s). | $C_{14}H_{11}N_2O_2F_3$ 296.26 | C 56.76 H 3.74 N 9.45 F 19.24 | 56.67 3.82 9.43 19.21 |
| 29 | 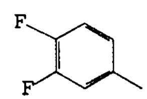 | 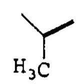 | 65 Méthode A | 155-6 | 3310(NH), 2212(CN), 1642, 1561, 1518, 1441, 1373, 1290. | $CDCl_3$ 15.78(1H,s); 7.84 (1H,s); 7.56 (1H,m); 7.16 (2H,m); 6.03 (1H,s); 5.69 (1H,m); 2.09(3H,t,J= 1.0 Hz). | $C_{13}H_{10}N_2O_2F_2$ 264.23 | C 59.09 H 3.81 N 10.60 F 14.38 | 58.91 3.85 10.59 14.41 |

TABLEAU I (suite)

| Ex | Ar | $R_1$ | Rendt % | F°C | IR $cm^{-1}$ | $^1H$ RMN δ | Formule M.wt | Analyse % Calc. | Trouvé |
|---|---|---|---|---|---|---|---|---|---|
| 30 | 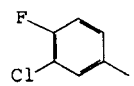 | 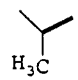 | 83 Méthode A | 198-200 | 3291(NH), 2212(CN), 1638, 1603, 1547, 1503, 1368. | $CDCl_3$ 15.73(1H,s); 7.71 (2H,m); 7.30 (2H,m): 6.05 (1H,s); 5.69 (1H,m); 2.09 (1H,s). | $C_{13}H_{10}N_2O_2$-ClF 280.68 | C 55.63<br>H 3.59<br>N 9.98<br>Cl12.63<br>F 6.77 | 55.47<br>3.63<br>9.96<br>12.89<br>6.70 |
| 31 | 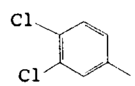 | 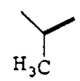 | 83 Méthode A | 181-3 | 3302(NH), 2220(CN), 1636, 1607, 1568, 1539, 1479, 1368, 1310, 953, 939. | $CDCl_3$ 15.71(1H,s); 7.82 (1H,s); 7.78(1H,d,J= 2.4 Hz); 7.44(1H,d,J= 8.6 Hz); 7.30(1H,dd); 6.05 (1H,s); 5.70 (1H,m); 2.09 (3H,s). | $C_{13}H_{10}N_2O_2$-$Cl_2$ 297.14 | C 52.55<br>H 3.39<br>N 9.43<br>Cl23.86 | 52.46<br>3.45<br>9.42<br>23.92 |
| 32 | 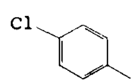 | 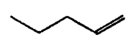 | 62 Méthode G | 138 | 3290(NH), 2220(CN), 1600, 1590, 1560, 1550, 1390, 1310, 1090, 1010, 830. | DMSO-$d_6$ 2.25-2.7 (4H,m); 4.95-5.17 (2H,m); 5.85(1H,mc); 7.38 and 7.58 (4H, AA'BB'); 10.6 (1H,s). | $C_{14}H_{13}N_2O_2Cl$ 262.72 | C 60.77<br>H 4.74<br>N 10.12<br>O 11.56<br>Cl12.81 | |
| 33 | 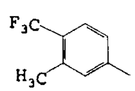 | 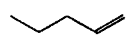 | 53 Méthode G | 133 | 3300(NH), 2220(CN), 1595, 1555, 1410, 1305, 1160, 1120, 1045, 840. | DMSO-$d_6$ 2.25-2.65 (7H,m); 4.95-5.15 (2H,m); 5.85(1H,m); 7.7-7.63 (3H,m); 11.3(1H,s). | $C_{16}H_{15}N_2O_2F_3$ 324.30 | C 59.26<br>H 4.66<br>N 8.64<br>O 9.87<br>F 17.57 | |

**EXEMPLE 34 :**

On a préparé des comprimés répondant à la formule suivante :

| | |
|---|---|
| - Composé de l'exemple 1<br>- Excipient q.s.p. pour un comprimé terminé à | 20 mg<br>150 mg |
| (Detail de l'excipient: lactose, amidon, talc, stéarate de magnésium). | |

**EXEMPLE 35 :**

On a préparé des comprimés répondant à la formule suivante :

| | |
|---|---|
| - Composé de l'exemple 2<br>- Excipient q.s.p. pour un comprimé terminé à | 20 mg<br>150 mg |
| (Detail de l'excipient: lactose, amidon, talc, stéarate de magnésium). | |

**ETUDE PHARMACOLOGIOUE**

Méthodes de tests biochimiques.

**1er test**

Oedème de la patte de rat (PO-R) induit par de la carragénine.

Une heure après l'administration orale des composés du test ou du véhicule témoin à des groupes de rats (n = 6-12, CFHB mâles, d'un poids entre 160-180 g), on injecte 1 mg de carragénine dissoute dans 0,2 ml de solution saline dans le coussinet digital de la patte arrière droite. Les pattes contralatérales reçoivent les injections témoin de solution saline. Les réactions d'oedèmes des pattes sont évaluées trois heures plus tard.

**2ème test**

Oedème à hypersensibilité de type retard de la patte de souris (DTH-M).

Des groupes de souris (n = 8-10), mâles CD-1, d'un poids entre 25-30 g) sont sensibilisés par une injection souscutanée de 1 mg de sérum-albumine bovine méthylé (MBSA) dans des volumes de 0,2 ml d'une solution saline/émulsion d'adjuvant complet de Freund (FCA). Des groupes témoin négatifs reçoivent des injections de solution saline/émulsion de FCA. Les réactions DHT d'oedème de la patte sont évaluées 24 heures après le défi dans le coussinet digital de la patte arrière droite avec 0,1 mg de MBSA dans des volumes de 0,05 ml de solution saline le 7ème jour après la sensibilisation. Les pattes contralatérales reçoivent des injections témoin de solution saline. Les composés du test ou les véhivules témoin sont administrés oralement une fois par jour les 4ème, 5ème et 6ème jour et deux fois par jour le 7ème jour, 1 heure avant et 6 heures après le défi au MBSA.

**3ème test**

Oedème à hypersensibilité de type retard de la patte de rat (DTH-R).

Des groupes de rats (n = 8-12, mâles CFHB, d'un poids entre 160-180 g) sont sensibilisés par une injection sous-cutanée à la base de la queue avec des volumes de 0,1 ml de FCA. Des groupes témoin négatifs reçoivent une injection d'adjuvant incomplet de Freund. Les réactions DTH d'oedème de la patte sont évaluées 24 heures après le défi dans le coussinet digital de la patte arrière droite avec 0,4 mg d'un extrait antigène de Mycobacterium tuberculosis dans des volumes de 0,2 ml de solution saline le 7ème jour après la sensibilisation. Les pattes contralatérales reçoivent des injections témoin de solution saline.

Les composés du test sont administrés oralement une fois par jour les 4ème, 5ème et 6ème jour et deux fois par jour le 7ème jour, 1 heure avant et 6 heures après le défi antigénique.

Les résultats de ces tests sont donnés dans le tableau II.
Les doses sont données en unités de mg/kg p.o.

## TABLEAU II

| Exemple | Test 1 | | Test 2 | | Test 3 | |
|---|---|---|---|---|---|---|
| | % inhibition | Dose | % inhibition | Dose | % inhibition | Dose |
| 1 | 31 | (50) | 54 | (100) | 78 | (50) |
| 2 | 16 | (50) | 7 | (100) | 65 | (50) |
| 3 | 30 | (50) | 36 | (100) | 20 | (50) |
| 4 | -24 | (50) | 45 | (100) | 4 | (50) |
| 5 | 23 | (50) | 50 | (100) | 16 | (50) |
| 6 | 30 | (50) | 75 | (30) | 79 | (50) |
| 7 | 43 | (50) | 62 | (100) | 65 | (50) |
| 8 | 23 | (50) | 60 | (100) | 92 | (50) |
| 9 | 12 | (50) | 44 | (100) | 64 | (50) |
| 10 | 24 | (50) | 61 | (100) | 41 | (50) |
| 11 | 24 | (50) | 26 | (100) | 33 | (10) |
| 12 | -3 | (10) | 43 | (30) | 43 | (10) |
| 13 | 24 | (50) | 62 | (100) | -10 | (50) |
| 14 | 14 | (10) | 66 | (30) | 28 | (10) |
| 15 | - | - | - | - | - | - |
| 16 | 8 | (50) | 17 | (100) | 62 | (50) |
| 17 | -24 | (50) | 15 | (100) | 60/39 | (50) |
| 18 | 15 | (50) | 19 | (100) | 9 | (50) |
| 19 | 39 | (50) | 55/62 | (100) | 24 | (50) |
| 20 | 7 | (50) | 47 | (100) | 26 | (50) |
| 21 | -5 | (50) | 16 | (100) | 30 | (50) |
| 22 | 11 | (50) | 16 | (30) | 78 | (50) |
| 23 | 22 | (50) | 23 | (30) | 62 | (50) |
| 24 | - | - | 64 | (30) | 78 | (50) |
| 25 | 24 | (50) | Toxic | (100) | 46 | (50) |
| 26 | 39 | (50) | Toxic | (100) | 54 | (50) |
| 27 | 14 | (50) | 6 | (100) | 7 | (50) |
| 28 | 39 | (50) | 20 | (100) | 13 | (50) |
| 29 | 39 | (50) | 18 | (100) | 34 | (50) |
| 30 | - | - | 52 | (100) | 37 | (50) |
| 31 | - | - | 53 | (100) | 66 | (50) |
| 32 | - | - | - | - | - | - |
| 33 | - | - | - | - | - | - |

**Revendications**

1. Nouveaux 2-cyano-3-hydroxy-ènamides répondant à la formule générale (I) :

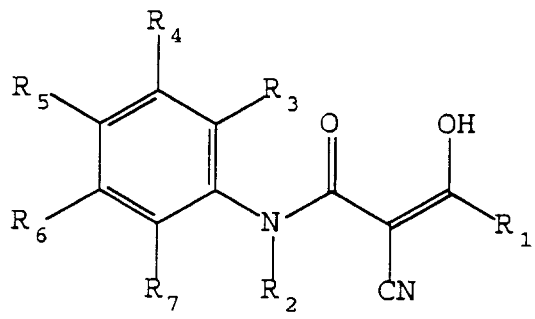

(I)

dans laquelle :

- $R_1$ représente un groupement de formule:

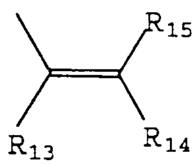

,

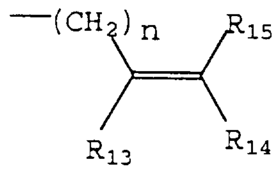

,

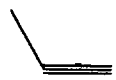

dans lequel $R_{13}$, $R_{14}$ ou $R_{15}$ représentent un atome d'hydrogène, un atome d'halogène, un radical alkyle renfermant de 1 à 3 atomes de carbone, n représente un nombre entier égal à 1, 2 ou 3,

- $R_2$ représente un atome d'hydrogène, un radical alkyle renfermant de 1 à 3 atomes de carbone,
- $R_3$, $R_4$, $R_5$, $R_6$ et $R_7$, identiques ou différents, représentent un atome d'hydrogène, un atome d'halogène, un groupement nitro ou cyano, un radical alkyle, linéaire ou ramifié, renfermant de 1 à 6 atomes de carbone,

un radical cycloalkyle renfermant de 3 à 6 atomes de carbone, un un radical alkoxy, linéaire ou ramifié, renfermant de 1 à 6 atomes de carbone, un radical alkylthio renfermant de 1 à 6 atomes de carbone, un radical $-(CH_2)_m-CF_3$, $-O-(CH_2)_m-CF_3$, $-S-(CH_2)_m-CF_3$ dans lesquels m représente un nombre entier compris entre 0 et 3,

un radical $-CF_2-Hal$, $-OCF_2-Hal$,

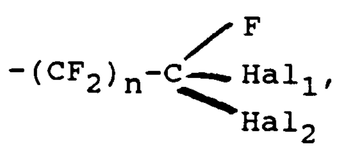

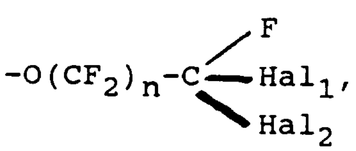

,

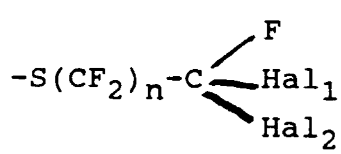

ou

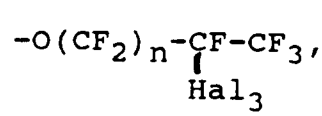

dans lequel n représente un nombre entier compris entre 1 et 3, Hal, $Hal_1$, $Hal_2$ et $Hal_3$, identiques ou différents, représentent un atome d'halogène,

ou $R_3$, $R_4$, $R_5$, $R_6$ et $R_7$, identiques ou différents, représentent un groupement de formule :

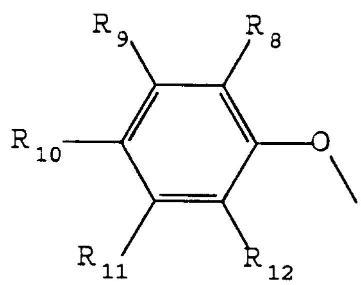

dans lequel $R_8$, $R_9$, $R_{10}$, $R_{11}$ et $R_{12}$, identiques ou différents, représentent l'une quelconque des valeurs indiquées ci-dessus pour $R_3$, $R_4$, $R_5$, $R_6$ et $R_7$ ou $R_4$ et $R_5$ forment ensemble un groupement $-O-CH_2-O$ et $R_3$, $R_6$ et $R_7$ ont la signification indiquée ci-dessus, leurs formes tautomères, ainsi que leurs sels d'addition avec les bases minérales ou organiques.

**2.** Nouveaux 2-cyano-3-oxo-ènamides, tels que définis par la formule (I) de la revendication 1, caractérisés en ce que dans ladite formule (I), $R_3$, $R_4$, $R_5$, $R_6$ et $R_7$, identiques ou différents, représentent un atome d'hydrogène, un atome de fluor, de chlore, de brome ou d'iode, un radical méthyle, éthyle, terbutyle, méthoxy, méthylthio, trifluorométhyle, trifluorométhoxy, trifluorométhylthio, pentafluoroéthyle, bromodifluorométhoxy, cyano, nitro, un groupement phénoxy, 4-chlorophénoxy ou $R_4$ et $R_5$ forment ensemble un groupement $-O-CH_2-O-$, $R_2$ représente un atome d'hydrogène, ou un radical méthyle, $R_1$, $R_3$, $R_6$ et $R_7$ ont la signification déjà indiquée, ainsi que leurs sels d'addition avec les bases minérales ou organiques.

**3.** Nouveaux 2-cyano-3-oxo-ènamides répondant à la formule (I) selon la revendication 1 ou 2, caractérisés en ce que dans ladite formule (I), $R_1$ représente un groupement :

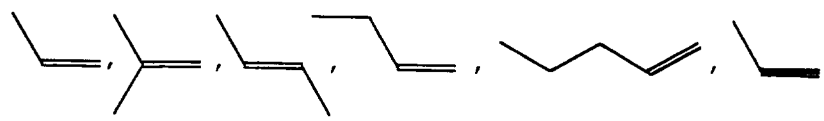

$R_2$ représente un atome d'hydrogène ou un radical méthyle, $R_3$, $R_4$, $R_5$, $R_6$ et $R_7$ identiques ou différents, représentent un atome d'hydrogène, un atome de fluor, de chlore ou de brome, un radical méthyle, trifluorométhyle, trifluorométhoxy, cyano, nitro ou un groupement phénoxy, ainsi que leurs sels d'addition avec les bases minérales ou organiques.

**4.** L'un quelconque des produits de formule (I), selon la revedication 1, dont les noms suivent :
2-cyano-3-hydroxy-4-méthyl-N-(4-trifluorométhylphényl)-penta-2,4-diénamide;
2-cyano-3-hydroxy-N-(4-trifluorométhylphényl)-hexa-2,5-diénamide;
2-cyano-3-hydroxy-4-méthyl-N-(4-chloro-3-trifluorométhylphényl)-penta-2,4-diénamide;
2-cyano-3-hydroxy-4-méthyl-N-(4-trifluorométhoxyphényl)-penta-2,4-diénamide;
2-cyano-3-hydroxy-4-méthyl-N-(4-bromophényl)-penta-2,4-diénamide;
2-cyano-3-hydroxy-N-(4-chloro-3-trifluorométhylphényl)-hexa-2,5-diénamide;
2-cyano-3-hydroxy-N-(3-méthyl-4-trifluorométhylphényl)-hepta-2,6-diénamide;
2-cyano-3-hydroxy-N-(4-trifluorométhylphényl)-hepta-2,6-diénamide;
ainsi que leurs sels d'addition avec les bases minérales ou organiques.

**5.** Procédé de préparation des nouveaux 2-cyano-3-oxo-ènamides tels que définis par la formule (I) de la revendication 1, ainsi que de leurs sels, caractérisé en ce que l'on fait réagir un produit de formule (II) :

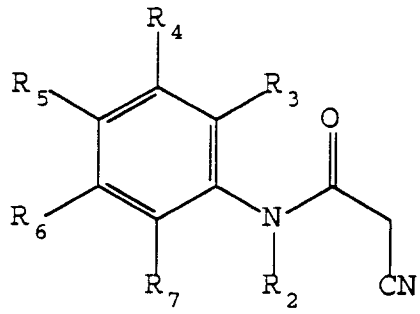

(II)

dans laquelle $R_2$, $R_3$, $R_4$, $R_5$, $R_6$ et $R_7$ ont la signification indiquée à la revendication 1, avec successivement de l'hydrure de sodium, si nécessaire en présence d'un catalyseur, puis :
soit avec le composé de formule (III):

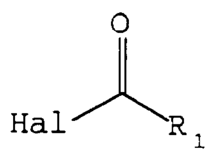

(III)

dans laquelle Hal représente un atome d'halogène et $R_1$ a la signification indiquée à la revendication 1,
b) ou avec un composé de formule ($III_A$):

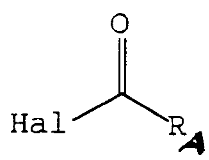

($III_A$)

dans laquelle Hal représente un atome d'halogène et $R_A$ représente un radical $R_1$ dûment protégé, pour obtenir un produit de formule ($I_A$) :

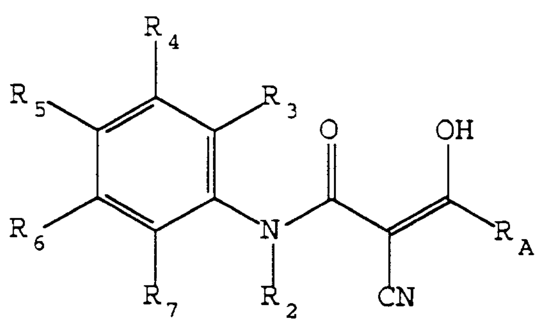

($I_A$)

dans laquelle $R_A$, $R_2$, $R_3$, $R_4$, $R_5$, $R_6$ et $R_7$ ont la signification déjà indiquée, puis, le cas échéant, déprotège le produit de formule ($I_A$) ainsi obtenu, pour obtenir un produit de formule (I) correspondant, que l'on isole et si désiré salifie.

**6.** Variante du procédé selon la revendication 5 pour la préparation des produits de formule (I) définie ci-dessus dans laquelle $R_1$ représente représente un groupe

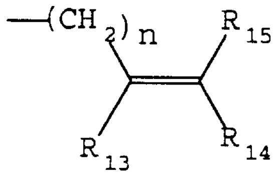

dans lequel $R_{13}$, $R_{14}$ et $R_{15}$ ont la signification indiquée à la revendication 1 et n a la valeur 2 ou 3, caractérisé en ce que l'on fait réagir un composé de formule (V):

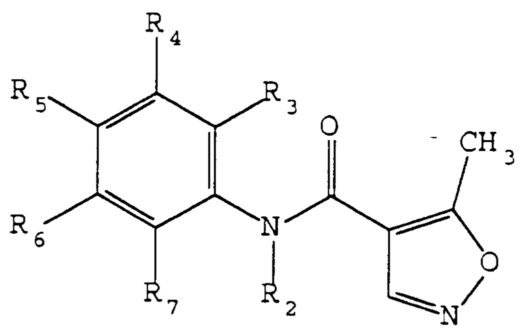

(V)

dans laquelle $R_2$, $R_3$, $R_4$, $R_5$, $R_6$ et $R_7$ ont la signification indiquée à la revendication 1 avec un composé de formule (VI)

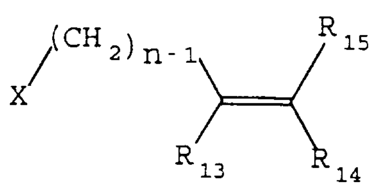

(VI)

dans laquelle X représente un groupement facilement éliminable et n, $R_{13}$, $R_{14}$ et $R_{15}$ ont la définition indiquée ci-dessus, en présence d'une base forte pour obtenir le produit de formule (I) correspondant que l'on isole et si désiré salifie.

**7.** Procédé de préparation selon la revendication 5, caractérisé en ce que :

- la réaction du produit de formule (II) avec l'hydrure de sodium est effectuée au sein d'un solvant organique anhydre tel que le tétrahydrofuranne, en présence d'un catalyseur tel que l'imidazole,
- la réaction avec le composé de formule (III) ou ($III_A$) est effectuée au sein d'un solvant organique tel que le tétrahydrofuranne à basse température,
- le radical $R_1$ peut être protégé par un groupement arylseleno tel qu'un groupement phénylseleno,
- la déprotection peut être effectuée par oxydation au moyen d'un péroxyde tel que le péroxyde d'hydrogène en l'absence de solvant ou bien au sein d'un solvant ou d'un mélange de solvants organiques tel que le mélange méthanol-dichlorométhane.

**8.** Procédé de préparation selon la revendication 6, caractérisé en ce que :

- dans le composé de formule (VI) , X représente un atome d'iode,
- la réaction du composé de formule (V) avec le composé de formule (VI) est effectuée dans un solvant organique tel que le tétrahydrofuranne, à basse température,
- la base forte utilisée peut être le butyllithium.

**9.** Médicaments, caractérisés en ce qu'ils sont constitués par les nouveaux 2-cyano-3-oxo-ènamides tels que définis par la formule (I) de la revendication 1, ainsi que par leurs sels d'addition avec les acides pharmaceutiquement acceptables.

**10.** Médicaments, caractérisés en ce qu'ils sont constitués par les nouveaux 2-cyano-3-oxo-ènamides tels que définis à l'une quelconque des revendications 2 à 4, ainsi que par leurs sels d'addition avec les acides pharmaceutiquement acceptables.

**11.** Compositions pharmaceutiques, caractérisés en ce qu'elles renferment à titre de principe actif, l'un au moins des médicaments tels que définis à l'une quelconque des revendications 9 ou 10.

## Claims

**1.** New 2-cyano-3-hydroxy-enamides corresponding to the general formula (I):

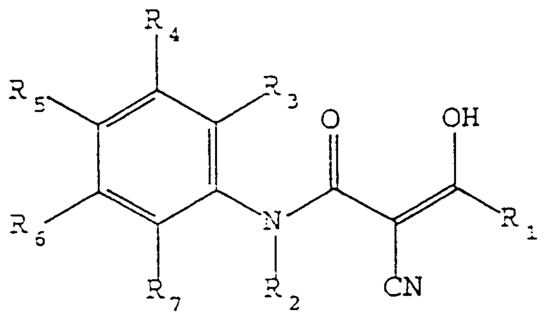

(I)

in which:

- $R_1$ represents a group of formula:

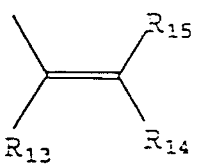 , 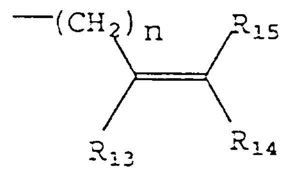 , 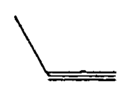

  in which $R_{13}$, $R_{14}$ or $R_{15}$ represents a hydrogen atom, a halogen atom, an alkyl radical containing 1 to 3 carbon atoms, n represents an integer equal to 1, 2 or 3,
- $R_2$ represents a hydrogen atom, an alkyl radical containing 1 to 3 carbon atoms,
- $R_3$, $R_4$, $R_5$, $R_6$ and $R_7$, identical or different, represent a hydrogen atom, a halogen atom, a nitro or cyano group, a linear or branched alkyl radical containing 1 to 6 carbon atoms, a cycloalkyl radical containing 3 to 6 carbon atoms, a linear or branched alkoxy radical containing 1 to 6 carbon atoms, an alkylthio radical containing 1 to 6 carbon atoms, a $-(CH_2)_m-CF_3$, $-O-(CH_2)_m-CF_3$, $-S-(CH_2)_m-CF_3$ radical in which m represents an integer comprised between 0 and 3,

one of the following radicals:
$-CF_2-Hal$, $-OCF_2-Hal$,

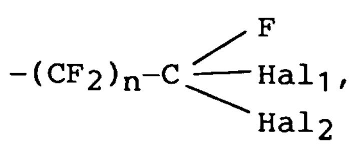

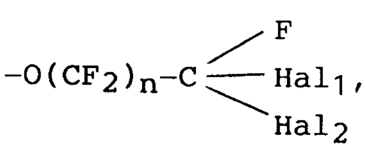 , 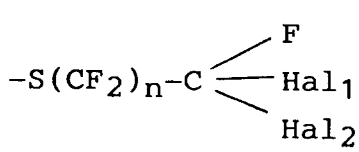 or 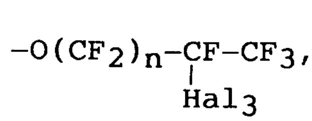

in which n represents an integer comprised between 1 and 3, Hal, $Hal_1$, $Hal_2$ and $Hal_3$, identical or different, represent a halogen atom,
or $R_3$, $R_4$, $R_5$, $R_6$ and $R_7$, identical or different, represent a group of formula:

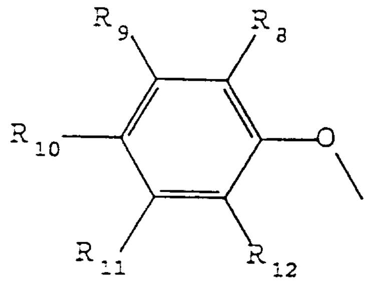

in which $R_8$, $R_9$, $R_{10}$, $R_{11}$ and $R_{12}$, identical or different, represent any one of the values indicated above for $R_3$, $R_4$, $R_5$, $R_6$ and $R_7$ or $R_4$ and $R_5$ together form an $-O-CH_2-O$ group and $R_3$, $R_6$ and $R_7$ have the meaning indicated above, their tautomeric forms, as well as their addition salts with mineral or organic bases.

**2.** New 2-cyano-3-oxo-enamides, as defined by formula (I) of claim 1, characterized in that in said formula (I), $R_3$, $R_4$, $R_5$, $R_6$ and $R_7$, identical or different, represent a hydrogen atom, a fluorine, chlorine, bromine or iodine atom, a methyl, ethyl, terbutyl, methoxy, methylthio, trifluoro-methyl, trifluoromethoxy, trifluoromethylthio, pentafluoro-ethyl, bromodifluoromethoxy, cyano, nitro radical, a phenoxy, 4-chlorophenoxy group or $R_4$ and $R_5$ together form an $-O-CH_2-O-$group, $R_2$ represents a hydrogen atom, or a methyl radical, $R_1$, $R_3$, $R_6$ and $R_7$ have the meaning already indicated, as well as their addition salts with mineral or organic bases.

**3.** New 2-cyano-3-oxo-enamides corresponding to formula (I) according to claim 1 or 2, characterized in that in said formula (I), $R_1$ represents a group:

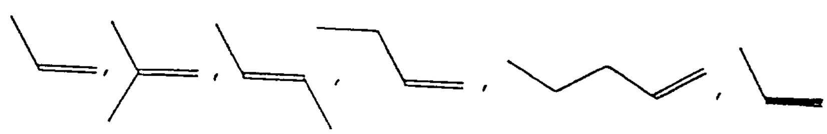

$R_2$ represents a hydrogen atom or a methyl radical, $R_3$, $R_4$, $R_5$, $R_6$ and $R_7$, identical or different, represent a hydrogen atom, a fluorine, chlorine or bromine atom, a methyl, trifluoromethyl, trifluoromethoxy, cyano, nitro radical or a phenoxy group, as well as their addition salts with mineral or organic bases.

**4.** Any one of the products of formula (I), according to claim 1, the names of which follow:
2-cyano-3-hydroxy-4-methyl-N-(4-trifluoromethylphenyl)-penta-2,4-dienamide;
2-cyano-3-hydroxy-N-(4-trifluoromethylphenyl)-hexa-2,5-dienamide;
2-cyano-3-hydroxy-4-methyl-N-(4-chloro-3-trifluoromethylphenyl)-penta-2,4-dienamide;
2-cyano-3-hydroxy-4-methyl-(4-trifluoromethoxyphenyl)-penta-2,4-dienamide;
2-cyano-3-hydroxy-4-methyl-N-(4-bromophenyl)-penta-2,4-dienamide;
2-cyano-3-hydroxy-N-(4-chloro-3-trifluoromethylphenyl)-hexa-2,5-dienamide;
2-cyano-3-hydroxy-N-(3-methyl-4-trifluoromethylphenyl)-hepta-2,6-dienamide;
2-cyano-3-hydroxy-N-(4-trifluoromethylphenyl)-hepta-2,6-dienamide;
as well as their addition salts with mineral or organic bases.

**5.** Preparation process for new 2-cyano-3-oxo-enamides as defined by formula (I) of claim 1, as well as for their salts, characterized in that a product of formula (II):

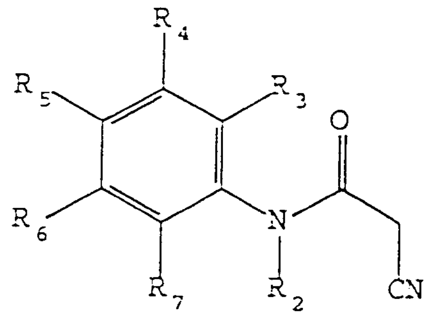

(II)

in which $R_2$, $R_3$, $R_4$, $R_5$, $R_6$ and $R_7$ have the meaning indicated in claim 1, is reacted successively with sodium hydride, if necessary in the presence of a catalyst, then:
either with the compound of formula (III):

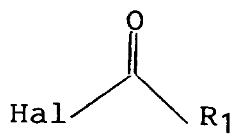

(III)

in which Hal represents a halogen atom and $R_1$ has the meaning indicated in claim 1,
b) or with a compound of formula ($III_A$):

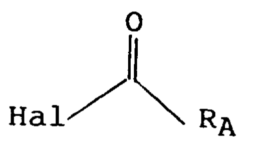

($III_A$)

in which Hal represents a halogen atom and $R_A$ represents a duly protected $R_1$ radical, in order to obtain a product of formula ($I_A$):

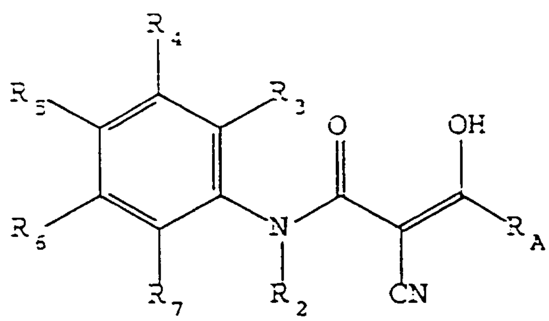

($I_A$)

in which $R_A$, $R_2$, $R_3$, $R_4$, $R_5$, $R_6$ and $R_7$ have the meaning already indicated, then, if appropriate, the product of formula ($I_A$) thus obtained is deprotected, in order to obtain a corresponding product of formula (I), which is isolated and if desired salified.

**6.** Variant of the process according to claim 5 for the preparation of the products of formula (I) defined above in which $R_1$ represents a group

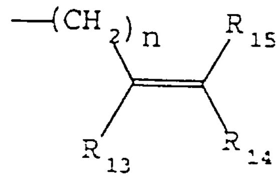

in which $R_{13}$, $R_{14}$ and $R_{15}$ have the meaning indicated in claim 1 and n has the value 2 or 3, characterized in that a compound of formula (V):

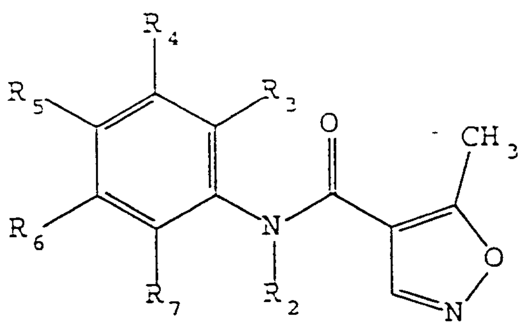

(V)

in which $R_2$, $R_3$, $R_4$, $R_5$, $R_6$ and $R_7$ have the meaning indicated in claim 1, is reacted with a compound of formula (VI):

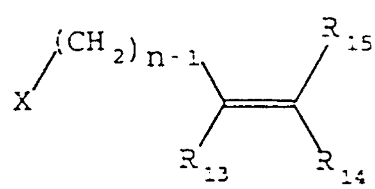

(VI)

in which X represents an easily eliminable group and n, $R_{13}$, $R_{14}$ and $R_{15}$ have the definition indicated above, in the presence of a strong base, in order to obtain the corresponding product of formula (I) which is isolated and if desired salified.

**7.** Preparation process according to claim 5, characterized in that:
- the reaction of the product of formula (II) with sodium hydride is carried out in an anhydrous organic solvent such as tetrahydrofuran, in the presence of a catalyst such as imidazole,
- the reaction with the compound of formula (III) or ($III_A$) is carried out in an organic solvent such as tetrahydrofuran at low temperature,
- the $R_1$ radical can be protected by an arylseleno group such as a phenylseleno group,
- the deprotection can be carried out by oxidation by means of a peroxide such as hydrogen peroxide in the absence of a solvent or in a solvent or a mixture of organic solvents such as a methanol-dichloromethane mixture.

**8.** Preparation process according to claim 6, characterized in that:
- in the compound of formula (VI), X represents an iodine atom,
- the reaction of the compound of formula (V) with the compound of formula (VI) is carried out in an organic solvent such as tetrahydrofuran, at low temperature,
- the strong base used can be butyllithium.

9. Medicaments, characterized in that they are constituted by the new 2-cyano-3-oxo-enamides as defined by formula (I) of claim 1, as well as by their addition salts with pharmaceutically acceptable acids.

10. Medicaments, characterized in that they are constituted by the new 2-cyano-3-oxo-enamides as defined in any one of claims 2 to 4, as well as by their addition salts with pharmaceutically acceptable acids.

11. Pharmaceutical compositions, characterized in that they contain as active ingredient, at least one of the medicaments as defined in any one of claims 9 or 10.

## Patentansprüche

1. Neue 2-Cyano-3-hydroxy-enamide entsprechend der allgemeinen Formel (I):

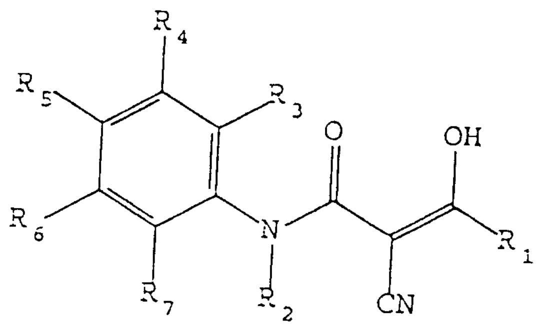

(I)

worin:
$R_1$ eine Gruppe der Formel

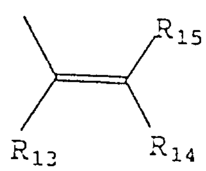
,
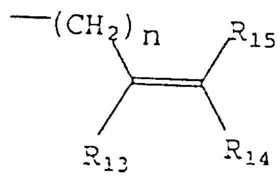
,
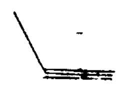

bedeutet, worin $R_{13}$, $R_{14}$ oder $R_{15}$ ein Wasserstoffatom, ein Halogenatom, einen Alkylrest mit 1 bis 3 Kohlenstoffatomen bedeuten, n eine ganze Zahl gleich 1, 2 oder 3 bedeutet,
$R_2$ ein Wasserstoffatom, einen Alkylrest mit 1 bis 3 Kohlenstoffatomen bedeutet,
$R_3$, $R_4$, $R_5$, $R_6$ und $R_7$, die identisch oder verschieden sind, ein Wasserstoffatom, ein Halogenatom, eine Nitro- oder Cyanogruppe, einen geraden oder verzweigten Alkylrest mit 1 bis 6 Kohlenstoffatomen, einen Cycloalkylrest mit 3 bis 6 Kohlenstoffatomen, einen geraden oder verzweigten Alkoxyrest mit 1 bis 6 Kohlenstoffatomen, einen Alkylthiorest mit 1 bis 6 Kohlenstoffatomen, einen Rest $-(CH_2)_m-CF_3$, $-O-(CH_2)_m-CF_3$, $-S-(CH_2)_m-CF_3$ bedeuten, worin m eine ganze Zahl zwischen 0 und 3 darstellt,
einen Rest $-CF_2$-Hal, $-OCF_2$-Hal,

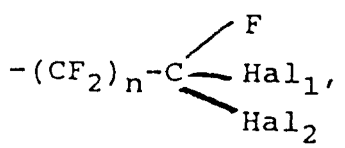

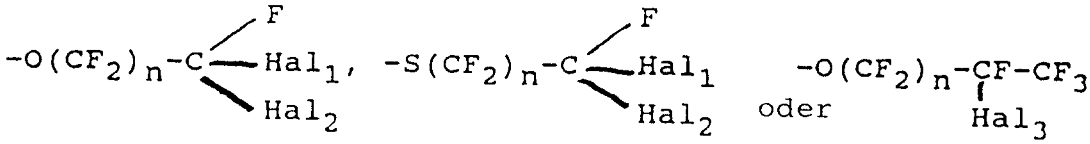

oder

worin n eine ganze Zahl zwischen 1 und 3 darstellt, Hal, $Hal_1$, $Hal_2$ und $Hal_3$, die identisch oder verschieden sind, ein Halogenatom bedeuten,
oder $R_3$, $R_4$, $R_5$, $R_6$ und $R_7$, die identisch oder verschieden sind, bedeuten eine Gruppe der Formel:

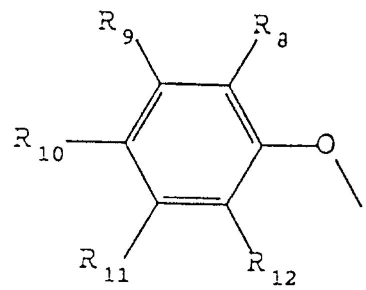

worin $R_8$, $R_9$, $R_{10}$, $R_{11}$ und $R_{12}$, die identisch oder verschieden sind, irgendeinen der oben für $R_3$, $R_4$, $R_5$, $R_6$ und $R_7$ angegebenen Werte bedeuten, oder $R_4$ und $R_5$ bilden zusammen eine Gruppe $-O-CH_2-O$ und $R_3$, $R_6$ und $R_7$ haben die oben angegebene Bedeutung,
ihre tautomeren Formen sowie ihre Additionssalze mit mineralischen oder organischen Basen.

2. Neue 2-Cyano-3-oxo-enamide, wie sie durch Formel (I) von Anspruch 1 definiert sind, dadurch gekennzeichnet, daß in dieser Formel (I) $R_3$, $R_4$, $R_5$, $R_6$ und $R_7$, die identisch oder verschieden sind, ein Wasserstoffatom, ein Fluor-, Chlor-, Brom- oder Jodatom darstellen, einen Methyl-, Ethyl-, tert-Butyl-, Methoxy-, Methylthio-, Trifluoromethyl-, Trifluoromethoxy-, Trifluoromethylthio-, Pentafluoroethyl-, Bromodifluoromethoxy-, Cyano-, Nitrorest, eine Phenoxygruppe, 4-Chlorophenoxy bedeuten,
oder $R_4$ und $R_5$ bilden zusammen eine Gruppe $-O-CH_2-O-$, $R_2$ bedeutet ein Wasserstoffatom oder einen Methylrest, $R_1$, $R_3$, $R_6$ und $R_7$ haben die bereits angegebene Bedeutung sowie ihre Additionssalze mit organischen oder mineralischen Basen.

3. Neue 2-Cyano-3-oxo-enamide entsprechend der Formel (I) gemäß Anspruch 1 oder 2, dadurch gekennzeichnet, daß in dieser Formel (I) $R_1$ eine Gruppierung

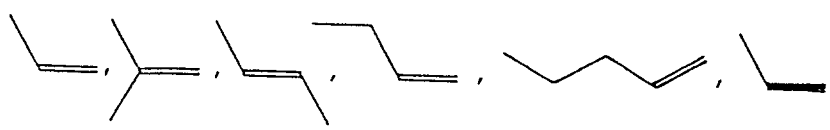

bedeutet,
$R_2$ ein Wasserstoffatom oder einen Methylrest darstellt,
$R_3$, $R_4$, $R_5$, $R_6$ und $R_7$, die identisch oder verschieden sind, ein Wasserstoffatom, ein Fluor-, Chlor- oder Bromatom, einen Methyl-, Trifluoromethyl-, Trifluoromethoxy-, Cyano-, Nitrorest oder eine Phenoxygruppe bedeuten, sowie ihre Additionssalze mit mineralischen oder organischen Basen.

4. Eines der Produkte der Formel (I) gemäß Anspruch 1 mit den folgenden Bezeichnungen:
2-Cyano-3-hydroxy-4-methyl-N-(4-trifluoromethylphenyl)-penta-2,4-dienamid;
2-Cyano-3-hydroxy-N-(4-trifluoromethylphenyl)-hexa-2,5-dienamid;
2-Cyano-3-hydroxy-4-methyl-N-(4-chloro-3-trifluoromethylphenyl)-penta-2,4-dienamid;
2-Cyano-3-hydroxy-4-methyl-N-(4-trifluoromethoxyphenyl)-penta-2,4-dienamid;
2-Cyano-3-hydroxy-4-methyl-N-(4-bromophenyl)-penta-2,4-dienamid;
2-Cyano-3-hydroxy-N-(4-chloro-3-trifluoromethylphenyl)-hexa-2,5-dienamid;

2-Cyano-3-hydroxy-N-(3-methyl-4-trifluoromethylphenyl)-hepta-2,6-dienamid;
2-Cyano-3-hydroxy-N-(4-trifluoromethylphenyl)-hepta-2,6-dienamid;
sowie ihre Additionssalze mit organischen oder mineralischen Basen.

**5.** Verfahren zur Herstellung der neuen 2-Cyano-3-oxo-enamide, wie sie durch Formel (I) von Anspruch 1 definiert sind, sowie ihrer Salze, dadurch gekennzeichnet, daß man ein Produkt der Formel (II):

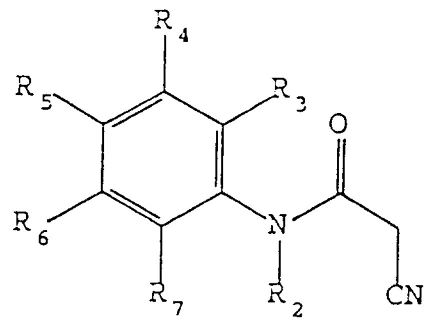

(II)

worin $R_2$, $R_3$, $R_4$, $R_5$, $R_6$ und $R_7$ die in Anspruch 1 angegebene Bedeutung haben, nacheinander mit Natriumhydrid, falls notwendig in Gegenwart eines Katalysators, zur Reaktion bringt, dann:
entweder mit der Verbindung der Formel (III):

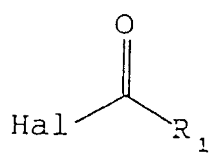

(III)

worin Hal ein Halogenatom bedeutet und $R_1$ die in Anspruch 1 angegebene Bedeutung hat,
b) oder mit einer Verbindung der Formel ($III_A$):

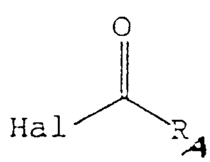

($III_A$)

worin Hal ein Halogenatom bedeutet und $R_A$ einen Rest $R_1$ entsprechend geschützt darstellt, um ein Produkt der Formel ($I_A$):

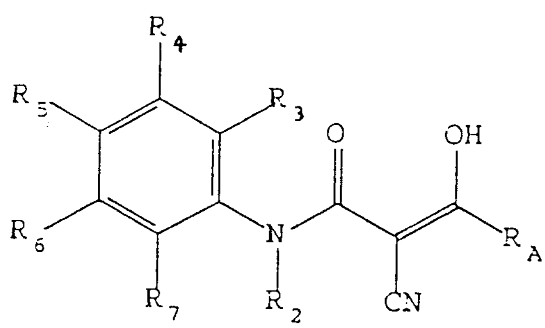

($I_A$)

zu erhalten, worin $R_A$, $R_2$, $R_3$, $R_4$, $R_5$, $R_6$ und $R_7$ die bereits angegebene Bedeutung haben,
dann gegebenenfalls in dem so erhaltenen Produkt der Formel ($I_A$) die Schutzgruppen abspaltet, um

ein entsprechendes Produkt der Formel (I) zu erhalten, das man isoliert und gewünschtenfalls in das Salz überführt.

**6.** Abänderung des Verfahrens gemäß Anspruch 5 zur Herstellung der Produkte der oben definierten Formel (I), worin $R_1$ eine Gruppe

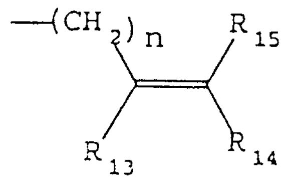

bedeutet, worin $R_{13}$, $R_{14}$ und $R_{15}$ die in Anspruch 1 angegebene Bedeutung und n den Wert 2 oder 3 hat, dadurch gekennzeichnet, daß man eine Verbindung der Formel (V):

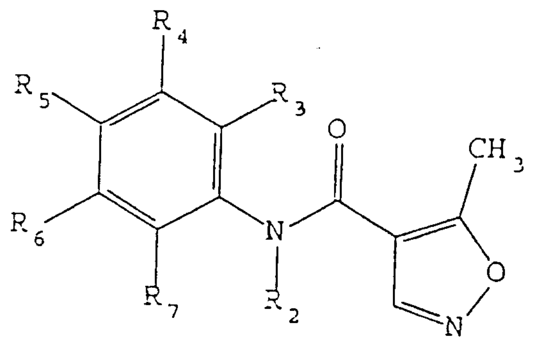

(V)

worin $R_2$, $R_3$, $R_4$, $R_5$, $R_6$ und $R_7$ die in Anspruch 1 angegebene Bedeutung haben, mit einer Verbindung der Formel (VI):

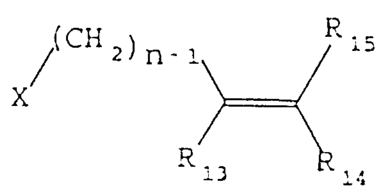

(VI)

worin X eine leicht entfernbare Gruppe bedeutet und n, $R_{13}$, $R_{14}$ und $R_{15}$ die oben angegebene Definition hat, in Gegenwart einer starken Base zur Reaktion bringt, um das entsprechende Produkt der Formel (I) zu erhalten, das man isoliert und gewünschtenfalls in das Salz überführt.

**7.** Herstellungsverfahren gemäß Anspruch 5, dadurch gekennzeichnet, daß:

- die Reaktion des Produkts der Formel (II) mit dem Natriumhydrid in einem wasserfreien organischen Lösungsmittel, wie Tetrahydrofuran, in Gegenwart eines Katalysators, wie Imidazol, durchgeführt wird;
- die Reaktion mit der Verbindung der Formel (III) oder ($III_A$) in einem organischen Lösungsmittel, wie Tetrahydrofuran, bei niedriger Temperatur durchgeführt wird;
- der Rest $R_1$ durch eine Arylselenogruppe, wie eine Phenylselenogruppe, geschützt werden kann;
- die Schutzgruppenentfernung durch Oxidation mit einem Peroxid, wie Wasserstoffperoxid, in Abwesenheit eines Lösungsmittels oder auch in einem Lösungsmittel oder einem Gemisch organischer Lösungsmittel, wie dem Gemisch Methanol/Dichlormethan, durchgeführt werden kann.

**8.** Herstellungsverfahren gemaß Anspruch 6, dadurch gekennzeichnet, daß:
- in der Verbindung der Formel (VI) X ein Jodatom bedeutet;
- die Reaktion der Verbindung der Formel (V) mit der Verbindung der Formel (VI) in einem organischen Lösungsmittel, wie Tetrahydrofuran, bei niedriger Temperatur durchgeführt wird;
- die verwendete starke Base Butyllithium sein kann.

**9.** Arzneimittel, dadurch gekennzeichnet, daß sie aus den neuen 2-Cyano-3-oxo-enamiden, wie sie durch Formel (I) von Anspruch 1 definiert sind, bestehen sowie aus ihren Additionssalzen mit pharmazeutisch annehmbaren Säuren.

**10.** Arzneimittel, dadurch gekennzeichnet, daß sie aus den neuen 2-Cyano-3-oxo-enamiden, wie sie in einem der Ansprüche 2 bis 4 definiert sind, bestehen sowie aus ihren Additionssalzen mit pharmazeutisch annehmbaren Säuren.

**11.** Pharmazeutische Zusammensetzungen, dadurch gekennzeichnet, daß sie als Wirkstoff mindestens eines der Arzneimittel, wie sie in einem der Ansprüche 9 oder 10 definiert sind, enthalten.